# EUROPEAN PATENT SPECIFICATION

(11) **EP 1 289 549 B1**
(45) Date of publication and mention of the grant of the patent: **10.12.2008**
(21) Application number: 01944823.2
(22) Date of filing: 15.06.2001
(51) Int. Cl.: A61K 39/39, A61K 39/385, A61K 39/00, A61P 37/04, C12N 15/62, C07K 19/00

(54) **THE USE OF PLANT OIL-BODIES IN VACCINE DELIVERY SYSTEMS**
VERWENDUNG VON PFLANZEN-ÖL-KÖRPER IN IMPFSTOFFVERABREICHUNGSYSTEME
UTILISATION DE CORPS LIPIDIQUES VEGETAUX DANS DES SYSTEMES D'ADMINISTRATION VACCINAUX

(30) Priority: 16.06.2000 US 212130 P
(43) Date of publication of application: 12.03.2003
(73) Proprietor: SemBioSys Genetics Inc., Calgary, AB T1Y 7L3 (CA)
(72) Inventor: SCHRYVERS, Anthony, B., Calgary, Alberta T3A 3V8 (CA); HUTCHINS, Wendy, A., Calgary, Alberta T2L 1Y3 (CA); MOLONEY, Maurice, M., Calgary, Alberta T3A 5N5 (CA); ALCANTARA, Joenel, Calgary, Alberta T2N 4M1 (CA)
(74) Representative: Williams, Aylsa
(86) International application number: PCT/CA2001/000872
(87) International publication number: WO 2001/095934

(56) References cited:
- WO-A-96/21029
- WO-A-98/53698
- US-A- 5 948 682

## Description

### FIELD OF THE INVENTION

The present invention provides a method for producing vaccines comprising plant oil-bodies and a desired antigen. The vaccines produced by the methods of the present invention can be used to elicit an immune response in an animal.

### BACKGROUND OF THE INVENTION

### Vaccine Development

There have been extensive efforts directed towards development of Subunit vaccines for human and veterinary disease control over the past two decades. Subunit vaccines are based on individual components derived from an infective agent that trigger the immune response. Identification of an appropriate antigen is only a first step in the development of a subunit vaccine as an effective adjuvant and delivery system as well as an economical means of production and purification of the desired antigen is required.

An adjuvant is any material that can increase the specific humoral and/or cellular response(s) to antigens. This rather broad definition has resulted in a highly heterogeneous collection of compounds being recognized as adjuvants. Thus it has been difficult to define a precise mode of action that is common to all adjuvants. It is widely believed that many adjuvants (i.e. emulsions, alum) act by forming antigenic deposits at the site of inoculation that slowly release antigens to cells of the immune system. The slow release of antigen results in a prolonged stimulation of the immune system for protracted periods. The particulate nature of the deposit may also enhance the uptake of antigen by the antigen processing cells, an important step for fully stimulating the immune system. In addition, some adjuvants contain components that stimulate the cells of the immune system and thus enhance the response to the antigen included in the formulation. More recently, molecular adjuvants are being developed that can stimulate specific cells or target antigens to specific cells and thus potentially have a more directed and predictable effect. Regardless of the exact mechanism, both cell-mediated and humoral immunity may be stimulated to varying degrees depending upon the antigen, the adjuvant, the protocol and the species involved.

The classic example of a highly effective adjuvant for eliciting a persistent immunological response after injection was described by J. Freund, (J. Immunol. 60:383-98, 1948). Freund's complete adjuvant is a combination of a mineral oil emulsion and killed mycobacteria. Although Freund's adjuvant and Freund's incomplete adjuvant (minus the mycobacteria) have been used extensively for immunization of laboratory animals for the production of antisera or immunological reagents, neither are acceptable for human clinical use because of side effects such as necrosis at the injection site. Other adjuvants that achieve a prolonged response are protein adsorbents such as aluminum hydroxide or aluminum phosphate. These substances provide a slow release but do not contribute to immunogenicity of the antigen itself.

Many of the known adjuvants can be grouped into one of four categories: (i) oil-based adjuvants, (ii) mineral-based adjuvants, (iii) bacterial products, or (iv) saponins and immunostimulating complexes. Oil-based adjuvants are prepared as water-in-oil or oil-in-water emulsions, commonly using pharmaceutical grade mineral oils that are nonmetabolizable. Freund's incomplete adjuvant is an example. The mineral-based adjuvants include aluminum hydroxide, aluminum phosphate and calcium phosphate. The ability of bacterial extracts to stimulate the immune system has been known for some time (i.e. mycobacterial extract in Freund's adjuvant). Several of the components that were responsible for immunostimulatory effects in bacterial extracts have been identified (i.e. muramyl dipeptide) and derivatives of these compounds have been developed in an attempt to reduce the undesired side effects when using these compounds. QuilA is an example of a saponin isolated from plants that has powerful immunostimulatory properties but can have adverse effects at higher doses. It has been included in a specifically formulated preparation of cholate and phospholipid to form what has been termed immunostimulating complexes (ISCOMs).

With the exception of ISCOMs, most of the conventional adjuvants are only useful for parenteral immunizations and alternative strategies had to been considered for enhancing mucosal immunizations. ISCOMs, biodegradable microspheres and liposomes are some examples of systems that have been developed and tested for mucosal immunization (Sjölander et al. J Leukocyte Biol 64:713-723,1998).

In order to develop a commercially viable and effective vaccine, the mass production of the selected antigenic substance and adjuvant delivery system must be cost effective. This situation is compounded by the fact that often more that one representative antigen (or more than one variant of an antigen) is required to provide adequate protection against the infective agent. Additionally, with an increasing number of specific vaccines being developed against different agents, there is a need for immunization with multiple antigens. This raises issues regarding compatibility of different antigens and vaccine formulations and significantly adds to the costs of developing vaccines. The potential for an increasing number of injections required for comprehensive immunization programs for children raises the additional concern that there may be reduced willingness to complete the entire series of injections, which in turn reduces efficacy of immunization programs. Thus it evident that alternative routes of administration more palatable to the vaccinee, particularly transdermal applications, would be ideal as a priming immunization, a booster immunization or perhaps as a complete replacement for parenteral immunizations.

The route of entry for most pathogenic organisms is via the mucosal surface and many infections are primarily localized to the mucosal and submucosal tissues. Advances in immunology have demonstrated that there is a sophisticated and integrated mucosal immune system capable of directing the immune response against potential pathogens. Conventional parenteral vaccines (injectables) are not very efficient for induction of a mucosal immune response. Considerable efforts have been directed towards developing systems for optimal mucosal immunization. Such approaches involve incorporation of the antigen into larger particles (liposomes, immunostimulating complexes, microspheres) that enhance delivery of antigen to the immune cells in the submucosal tissues. The mucosal route of administration is attractive to patients and has even prompted consideration of mucosal immunization for systemic infections. However, although mucosal immunizations may be efficacious in some circumstances, recent studies indicate that a combination of both mucosal and parenteral immunization is required for optimal induction of an effective immune response for many infections. At present, vaccine formulations for systemic and mucosal administration are distinct and thus the expense of developing separate formulations for this immunization strategy is a potential barrier for cost-effective implementation.

The skin is an effective barrier to pathogens and thus provides a level of protection against infections. However, if pathogens manage to gain entry through the dermal layer, there are a variety of immunologically active cells (i.e. dendritic cells) present in the skin that are capable of initiating an effective immune response. This is the basis for the subdermal parenteral immunizations that have been routinely used clinically in humans and experimentally in animals. However, the barrier function of the skin has also served as a barrier to development of transdermal immunization strategies. Although there is a rather extensive scientific and patent literature on means of delivery of small molecule therapeutics through the skin, there is little literature on transdermal (transcutaneous) immunizations. Unlike most protein antigens, transcutaneous application of certain bacterial toxins can result in induction of an immune response (Glenn et al. Infect Immunity 67:1100-1106, 1999), but no general delivery system for protein antigens has been described. Clearly there would be many advantages to a system involving application of an antigen-containing preparation to the surface of the skin and effectively delivering the antigens to the intradermal dendritic cells.

Recently there have been tremendous advances in our understanding of the molecular and cellular aspects of the immune response. This provides the opportunity for enhancing and directing the immune response appropriately against particular infectious agents. Thus it is possible to primarily induce an antibody (humoral, Th2) response against extracellular pathogens or direct the immune response to cell-mediated (Th1) mechanisms for intracellular pathogens. Studies have demonstrated that several of the molecular adjuvants are more efficacious if they are physically linked to the antigen as the adjuvant effect may rely on effective targeting and delivery of antigen to the appropriate antigen processing cells. In addition, incorporating targeting molecules into delivery vehicles, such as liposomes, can provide an adjuvant effect to the associated antigens (Harokopakis et al., J. Immunol. Methods 185:31-42, 1995). However, incorporating molecular adjuvants into vaccines may result in considerable additional costs due to the production and/or purification of the component and optimal incorporation into the vaccine formulation (i.e. conjugation).

In consideration of the information described above it is apparent that an ideal vaccine would include multiple antigens, be amenable to transdermal, mucosal or systemic administration and would incorporate components to modulate and focus the immune response appropriately. The plant oil-body system provides a simple and inexpensive means of producing complex multiple vaccine formulations and thus provides a means to develop improved vaccine formulations at acceptable cost.

### Plant Oil-bodies

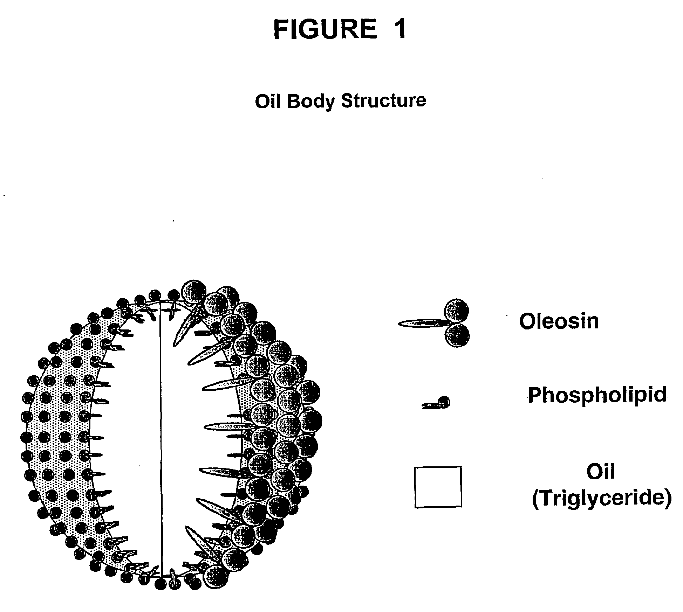

Oil-bodies are the organelles in which triacylglycerides (oil; neutral lipids) are stored in plant seeds. Plant oil-bodies are spherical particles about 0.5 - 2 m in diameter and are found in all oilseed plants (i.e. Canola, flax, sunflower, soybean and corn). The oil-bodies consist primarily of central droplet of oil (triglycerides) that is surrounded by a coat consisting of a phospholipid monolayer and a protein called oleosin. The general structure of a plant oil-body is illustrated in Figure 1. Oleosin and the oil-bodies are almost exclusively made during seed development and accumulate to very high levels in the seeds of oil seed plants. Oil-bodies are approximately the size of a small bacterium and thus would tend to be processed by the immune system (i.e. phagocytosis by antigen processing cells) in a similar fashion. The composition and structure of oil-bodies to some degree parallels that of synthetic vaccine adjuvant/delivery systems being considered by the vaccine industry (Garcon and Six 1991; Sjölander, Cox *et al.* 1998) and thus could serve as a natural adjuvant/delivery system. Due to their size, oil-bodies could be readily sampled by mucosal (M) cells and effectively delivered to the cells that constitute the mucosal immune system. Oil-bodies are stable structures and are of an appropriate size to be effectively aerosolized for delivery to the nasal or pulmonary mucosa. Concentrated suspensions of oil-bodies have the consistency of a cream or lotion and thus may be convenient for applying to the surface of the skin. Oil-bodies can be isolated in large quantities from many plant species and appear to be relatively stable structures once isolated.

### Plant Oils as Adjuvants

There is a substantial patent literature on the use of oils in adjuvants to potentiate immunity, including ones utilizing oils derived from plant seeds. The majority of these patents disclose formulations and uses of water-in-oil or oil-in-water emulsions, which are prepared by mixing chemically pure compositions. The primary advantage of plant-derived oil-based adjuvants over the mineral oil-based adjuvants is that they are metabolizable and thus are likely to be better tolerated. However, this may also result in a reduced efficacy as an adjuvant. There are even some patents, such as US patent No. 4,125,603 by Audibert et al*.,* which include a nonimmunogenic protein (bovine serum albumin) during emulsion formation to act as a stabilizer. However, this patent does not describe a composition that truly resembles that of plant oil bodies, or would provide the uniform structural features that are characteristic of plant oil bodies. PCT Patent application WO 98/53698 describes the use of oil bodies in various food products, personal care products and pharmaceutical products. PCT Patent application WO 00/30602 discloses the use of oil bodies as topical delivery vehicles for active agents, for example cosmetic compounds and therapeutic actives in general. To the best of our knowledge, none of the existing patent literature describes the use of the oil bodies directly as an adjuvant system.

### Planet Protein Production Systems

Plants offer an attractive alternative to animal, insect and cell culture platforms for the large-scale production of recombinant proteins of commercial value. Interest in plant production of compounds, often referred to as "Molecular Farming" has increased dramatically in the past few years and many different production platforms have been described. Plants offer the potential for large scale, comparatively inexpensive production that is safe and reliable. Although the production of any number of different commercially valuable proteins can be achieved in plants, the manufacture of proteins that function as antigens is of significant interest because of the commercial value of vaccines and the potential convenience of making plant based vaccines that can be consumed by animals and humans as part of their diet.

The production of foreign proteins of commercial value in plants is first described in a family of patents by Goodman *et al.,* (US 4,956,282; US 5,550,038; US; US 5,629,175, EP233915, CA1340696), that describe mammalian peptide expression in plant cells. The claims are directed to the production of mammalian peptides in dicot species; however, the potential production of proteins that would serve as antigens in the formation of vaccines is described in the specification. Although these patents suggest that antigens, (e.g. envelope proteins of leukemia and lymphotropic retroviruses, surface antigens of herpes simplex virus or hepatitis B virus), could be manufactured in plants, no examples are provided demonstrating the production and efficacy of such a plant based vaccine. Additionally the method described would involve complex and expensive purification procedures in order to isolate sufficient antigens that could be used in vaccine formulation. These patents describe only production of a peptide that could be an antigen, but do not include direction to provide stimulation of the immune response with an added adjuvant.

Further examples of the production of foreign proteins in plants include: US 5,487,991 by J. S. Vandekerckhove et al*.* entitled "A Process for the Production of Biologically Active Peptide Via the Expression of Modified Storage Seed Protein Genes in Transgenic Plants". This invention describes the production of seed storage protein and a foreign protein of interest joined in-frame. Provision is made for proteolytic cleavage sequences at the junctures of these two proteins such that the amino acid sequence of commercial interest can be cleaved free of the storage protein. The invention as described is proposed to be an improvement for levels of foreign protein expression, stability of the product and ease of recovery. The limitation of the approach, however, is the accommodation of foreign sequences within fusion proteins without disturbing the three dimensional structure and hence storage capacity and stability. The size of the peptides that can be accepted is small and the specification does not expressly recite production of antigenic peptides and vaccines.

Three related patents, US 5,654,184, US 5,679,880, US 5,686,079 by R. Curtiss and G. A. Cardineau, entitled "Oral Immunization by Transgenic Plants" describe the commercial exploitation of plants as delivery vehicles for antigens and use as oral vaccines for immunization. However, this approach is restricted to oral immunization and thus is faced with potential problems of stability of antigens during transit through the stomach.

Additional related patents include " Production of Enzymes in Seeds and Their Use" by J. J. van Ooijen *et al*., US 5,543,576, US 5,714,474, which describes a method of catalyzing reactions using seeds containing recombinant enzymes. The crushed seeds are used directly and as no attempt is made to recover or purify the enzyme. This invention does not contemplate the production of antigenic substances. Other patents, (US 5,650,307, US 5,716,802, US 5,763,748,) by P. C. Sijmons et al*.,* entitled: "Production of heterologous proteins in plants" also describe methods for the isolation of heterologous proteins in plants. These inventions teach production of proteins in plants using the ability of plants to process and excrete proteins from the plant cell. The basic process involves the use of a leader sequence which the plant recognizes and hence directs the recombinant protein to the extracellular space. In particular the methods describe the tailoring the excretion of the protein in a plant species to blend with an industrial process, such as starch processing. The invention is exemplified by the production of human serum albumin (HSA) in potato.

A method of producing valuable proteins in plants by exploiting the synthetic capacity of the aleurone tissue of cereals is the subject of a patent entitled " Producing Commercially Valuable Polypeptides with Genetically Transformed Endosperm Tissue", US 5,677,474 by J. C. Rogers. This patent relates specifically to the heterologous production of proteins in cereals but does not consider the production of antigens and the formation of vaccines.

Technology directed to production of antibodies in plants are disclosed in US 5,202,422, US 5,639,947, US 5,959,177 by Hein et al*.* These patents describe the use of plants to express antibodies that are multimeric proteins. Such plants are then used in a method to produce passive immunity to a pathogen by administration of said immunoglobulins.

The concept that transgenic plants can be used as a vehicle to deliver oral vaccines is described in issued US patents, (US 5,484,719; US 5,612,487; US 5,914,123, US 6,034,298) authored by D. Lam and C. Arntzen. These patents describe the transformation of plants with a DNA sequence encoding the expression of a surface antigen of a viral pathogen ligated to a promoter that expresses in plant cells. The preferred embodiment of the invention is the expression in a portion of the plant that is edible by humans or animals such as a fruit or juice that can be taken orally. This approach may be useful for antigens that are suitable for oral immunization such as some bacterial toxins (cholera toxin beta subunit) or viral particles but is unlikely to be suitable for a wide range of antigens. In addition, in practice it will be difficult to control the amount of antigen delivered to the mucosal immune system and to completely avoid undesirable outcomes such as development of oral tolerance.

A method of producing recombinant proteins of interest in the latex fluid of the rubber plant is described in US 5,580,768 by Boffey et al*.,* entitled " Method for Production of Proteins in Plant Fluids". By the use of a latex specific promoter the production of recombinant proteins of commercial value could be made by rubber trees and harvested conveniently by established simple methods. The specification suggests that any number of pharmaceutically important proteins could be manufactured by such a method, however the production of antigenic proteins is not disclosed.

A heterologous protein production system based on the expression of foreign genes under the control of a promoter that is induced by injury or other mechanical stimuli is the subject of patents (US 5,670,349 and US 5,689,056) by C. L. Cramer and D. L. Weissenborn, entitled "HMG2 Promoter Expression System and Post-harvest Production of Gene Products in Plants and Plant Cell Cultures". The HMG2 promoter elements are responsive to pathogen infection, pest-infestation, wounding, elicitor and chemical treatments. These promoter elements, which may be derived from numerous different species of plants, can be used to drive the expression of a variety of different exogenous genes including proteins of medicinal value. The applications of the invention that have been exemplified relate to the production of enzymes. A further patent, US 5,929,304, Radin *et al.,* "Production of lysosomal enzymes in plant-based expression systems" was issued recently.

Another family of recently issued patents describes a two-step process for producing recombinant proteins in cereal seeds and cell cultures from cereal plants. US 5,693,506, US 5,888,789, US 5,889,189, US 5,994,628, entitled " Process for Protein Production in Plants" were authored by R. L. Rodriguez. Genes encoding recombinant proteins are inserted into two separate expression constructs. One construct uses a promoter active during germination or malting of seed. The other construct employs a hormonally regulated promoter to achieve expression in the malted seed. In addition both constructs employ regulatory sequences that allow the target protein to be excreted. The invention is exemplified by GUS expression driven by the alpha amylase promoter in malted rice, examples of production of an antigen or vaccine are provided.

Other patents that describe heterologous protein production in plants include: US 5,723,755, " Large Scale Production of Human or Animal Proteins Using Plant Bioreactors " by Marc Fortin, US 5,990,385, entitled " Protein production in transgenic alfalfa plants issued to Vezina et al., US 5,824,870, "Commercial production of aprotinin in plants, by Baszczynski et al.,; US 5,767,379, "Commercial production of avidin in plants, by Baszczynski et al., and US 5,804,694, "Commercial production of β - glucuronidase in plants", by *Bruce et al.*

In summary numerous protein production platforms for heterologous production of proteins of commercial and medicinal value have been described in the literature and in issued patents. Some of these inventions expressly recite the production of proteins that are antigenic and potentially may be used in the formulation of vaccines. In particular inventions have been disclosed whereby transformed plant tissues or cells expressing the antigenic protein may be consumed directly as edible vaccines. The practical limitations of the systems described above, however, originate in the modest levels of heterologous proteins that may be produced or the cost and difficulty of recovery of the protein in a pure form suitable for inclusion in a conventional vaccine. None of the systems so described naturally include adjuvants required to stimulate and prolong the immunogenic response.

The subject of the present invention is vaccine delivery system comprised of plant oil bodies that contain antigens that are displayed on the oil body surface. One embodiment of the invention involves a protein production platform derived from plant oil bodies in which the antigen is expressed as a fusion protein with the major oil body surface protein, oleosin. The production of chimeric heterologous proteins in association with plant oil bodies is described in patents authored by M. Moloney: US 5,650, 554, " Oil-body proteins as carriers of high value peptides in plants"; US 5,792,922, " Oil-body protein cis-elements as regulatory signals; US 5,856,452, "Oil bodies and associated proteins as affinity matrices"; US 5,948,682, " Preparation of heterologous proteins on oil bodies.

Oleosin proteins comprise 2-10% of total cellular protein and are tightly associated with the oil-bodies of developing seed cells (Huang, A. H. C., Ann Rev Plant Physiol and Plant Mol Biol 43:177-200, 1992; Murphy, D. J., Prog. Lipid Res 29:229-324, 1991). Unlike other cellular proteins, oleosins partition with the oil fraction of a seed cell extract. Thus by floatation centrifugation, or even on standing, oleosins are easily separated from all other cellular proteins (Parmenter, et al., Plant Mol. Biol. 29:1167-1180, 1995). Studies have demonstrated that any protein that is physically attached to the oil-body, particularly proteins covalently bound to oleosins, will separate with the oil-body fraction (van Rooijen, G. J. H., and Moloney, M. M., Bio/Technology 13:72-77, 1995; van Rooijen, G. J. H., and Moloney, M. M. Plant Physiol. 109:1353-1361, 1995). This provides an efficient mechanical mechanism for purification of a recombinant protein. In consideration of the economics of plant production, this provides a very inexpensive means of protein production.

The following publications are representative of the state of the art.
Garcon, N. M. J. and H. R. Six. 1991. Universal vaccine carrier: Liposomes that provide T-dependent help to weak antigens. J.Immunol. 146: 3697-3702.
Glenn, G. M. et al. 1999. Trancutaneous immunization with bacterial ADP-ribosylating exotoxins as antigens and adjuvants. Infection and Immunity 67(3): 1100-1106.
Harokopakis, E. et al. 1995. Conjugation of cholera toxin or its B subunit to liposomes for targeted delivery of antigens. J.Immunol.Methods 185: 31-42.
Issartel, J.-P. et al. 1991. Activation of Escherichia coli prohaemolysin to the mature toxin by acyl carrier protein-dependent fatty acylation. Nature 351: 759-761.
Sjölander, A., et al. 1998. ISCOMs: an adjuvant with multiple functions. J.Leukocyte Biol 64: 713-723.
Wu, H. C. and M. Tokunaga, 1986. Biogenesis of lipoproteins in bacteria. Curr.Top.Microbiol.Immunol. 125: 127-157.
Chen, J.C. et al., 1999, Cloning and secondary structure analysis of caleosin, a unique calcium-binding protein in oil bodies of plant seeds. Plant Cell Physiol 40:1079-86
Bechtold, N. et al., 1993, In planta Agrobacterium-mediated gene transfer by infiltration of adult plants. C.R. Acad. Sci. Paris, Life Sciences 316:1194-1199
Danve, B., Lissolo, L., Guinet, F., Boutry, E., Speck, D., Cadoz, M., Nassif, X., Quentin-Millet, M. J. Safety and immunogenicity of a Neisseria meningitidis group B transferrin binding protein vaccine in adults. Nassif, X., Quentin-Millet, M.-J., and Taha, M.-K. 53. 98. (Eleventh International Pathogenic Neisseria Conference).
Freund, J.,1948, J. Immunol. 60:383-98.
Harland, R. J. et al. 1992. The effect of subunit or modified live bovine herpesvirus-1 vaccines on the efficacy of a recombinant Pasteurella haemolytica vaccine for the prevention of respiratory disease in feedlot calves. Can. Vet. J. 33:734-741.
Halpern, J. L. et al. 1990. Cloning and expression of functional fragment C of tetanus toxin. Infect Immun. 58:1004-1009.
Harokopakis, E., et al. 1995, Conjugation of cholera toxin or its B subunit to liposomes for targeted delivery of antigens., J. Immunol. Methods 185:31-42.
Huang, A. H. C., 1991, Oil-bodies and oleosins in seeds, Ann Rev Plant Physiol and Plant Mol Biol 43:177-200,
Kuhnel, B., et al. 1996. Oil bodies of transgenic Brassica napus as a source of immobilized b-glucuronidase. JAOCS 73:1533-1538.
Murphy, D. J.,1991, Structure, function and biogenesis of storage lipid bodies and oleosins in plants, Prog, Lipid Res. 29:299-324.
Parmenter, D.L., et al. 1995, Production of biologically active hirudin in plant seeds, Plant Mol. Biol. 29:1167-1180.
Riggs, P. 1994. Expression and purification of maltose binding protein fusions., p. 16-6-1-16-6-14. In: F. M. Ausubel, R. Brent, R. E. Kingston, D. D. Moore, J. G. Seidman, J. A. Smith, and K. Struhl (eds.), Current Protocols in Molecular Biology. Wiley, New York.
Schantz, P. J.,1993, Use of peptide libraries to map the substrate specificity of a peptide-modifying enzyme: A 13 residue consensus peptide specifies biotinylation in Escherichia coli. Bio/Technology 11:1138-1143),
Tsao, K. L., et al. 1996. A versatile plasmid expression vector for the production of biotinylated proteins by site-specific, enzymatic modification in Escherichia coli., Gene 169:59-64.
van Rooijen, G. J. H., and Moloney, M. M., 1995, Plant seed oil-bodies as carriers for foreign proteins, Bio/Technology 13:72-77
van Rooijen, G. J. H., and Moloney, M. M., 1995, Structural requirements of oleosin domains for subcellular targeting to the oil body, Plant Physiol. 109:1353-1361.
Arntzen, C. J., et al. US 5,914,123, Vaccines expressed in plants.
Baszczynski, C., et al. US 5,824,870, Commercial production of aprotinin in plants.
Baszczynski, C., et al. US 5,767,379, Commercial production of avidin in plants.
Boffey, S. A., et al. US 5,580,768, Method for the production of proteins in plant fluids.
Bruce, W. B., et al. US 5,804,694, Commercial production of B-glucuronidase in plants.
Cramer, C. L., et al. US 5,670,349,HMG2 promoter expression system and post-harvest production of gene products in plants and plant cell cultures. Cramer, C. L., et al. US 5,689,056, HMG2 promoter expression system
Curtiss, R. III., et al. US 5,654,184, Oral immunization by transgenic plants.
Curtiss, R. III., et al. US 5,679,880, Oral immunization by transgenic plants.
Curtiss, R. III., et al. US 5,686,079, Oral immunization by transgenic plants.
Fortin, M. G., US 5,723,755, Large scale production of human or animal proteins using plant bioreactors.
Goodman, R. M., et al. US 4,956,282, Mammalian peptide expression in plant cells.
Goodman, R. M., et al. EP233915B1, Feb 3,1993, Molecular Farming.
Goodman, R. M., et al. US 5,550,038, Molecular Farming.
Goodman, R. M., et al. US 5,629,175, Molecular Farming.
Hein, M. B., et al. US 5,959,177, Transgenic plants expressing assembled secretory antibodies.
Hiatt, A. C., et al. US 5,202,422, Compositions containing plant-produced glycopolypeptide multimers, multimeric proteins and method of their use.
Hiatt, A. C., et al. US 5,639,947, Compositions containing glycoprotein multimers and methods of making same in plants.
Knauf, V. C., et al. CA1340696, Aug 10, 1999, Mammalian peptide expression in plant cells.
Lam, D. M., et al. US 5,484,719, Vaccines produced and administered through edible plants.
Lam, D. M., et al. US 5,612487, Anti-viral vaccines expressed in plants..
Lam, D. M., et al. US 6,034,298, Vaccines expressed in plants.
Moloney, M. M., US 5,650,554, Oil-body proteins as carriers of high-value peptides in plants.
Moloney, M. M., US 5,792,922, Oil-body protein cis-elements as regulatory signals.
Moloney, M. M., US 5,948,682, Preparation of heterologous proteins on oil bodies.
Moloney, M. M., et al. US 5,188,958, Transformation and foreign gene expression in *Brassica* species.
Moloney, M. M., et al. US 5,463,174, Transformation and foreign gene expression in *Brassica* species.
Moloney, M. M., et al. US 5,750,871 Transformation and foreign gene expression in *Brassica* species.
Moloney, M. M., et al. US 5,856,452, Oil bodies and associated proteins as affinity matrices.
Radin, D. N., et al. US 5,929,304, Production of lysosomal enzymes in plant-based expression systems.
Rodriguez, R. L., US 5,693,506, Process for protein production in plants,
Rodriguez, R. L., US 5,888,789, Process for protein production in plants.
Rodriguez, R. L., US 5,889,189, Process for protein production in plants.
Rodriguez, R. L., US 5,994,628, Process for protein production in plants.
Rogers, J. C., US 5,677,474, Oct 14, 1997, Producing commercially valuable polypeptides with genetically transformed endosperm tissue.
Schilperoort, R. A., et al. US 4,940,838, Process for incorporation of foreign DNA into the genome of dicotyledonous plants.
Schilperoort, R. A., et al. US 5,464,763, Process for the incorporation of foreign DNA into the genome of dicotyledonous plants.
Sijmons, P. C., et al. US 5,650,307, Production of heterologous proteins in plants and plant cells.
Sijmons, P. C., et al. US 5,716,802, Production of heterologous proteins in plants and plant cells.
Sijmons, P. C., et al. US 5,763,748, Production of heterologous proteins in plants and plant cells.
Van Ooijen, A. J. J., et al. US 5,543,576, Production of enzymes in seeds and their use.
Van Ooijen, A. J. J., US 5,714,474, Production of enzymes in seeds and their use.
Vandekerckhove, J.S., et al. US 5,487,991, A Process for the production of biologically active peptide via the expression of modified storage seed protein genes in transgenic plants.
Vezina, L-P., et al. US 5,990,385, Nov 23,1999, Protein production in transgenic alfalfa plants.

### SUMMARY OF THE INVENTION

The subject method provides a means for the production of vaccines comprising plant oil-bodies and a desired antigen. The use of a plant oil-body provides a convenient and safe adjuvant that enhances the immune response to the antigen and eliminates the need for additional adjuvants. The method allows for the production of vaccine compositions containing one or more antigens that can be administered by a variety of means including injection, as well as vaccines that can be administered transdermally or through the mucosa. The method further allows for the production of vaccines where the antigen is produced as part of a plant oil-body through the fusion of the antigen to an oil-body protein and expression in a recombinant plant host. The method also allows for production of a vaccine composition comprising oil-bodies and an antigen coupled to the oil-body by chemical means.

A method of eliciting an immune response in an animal, wherein said method comprises administering a formulation comprising an oil body and an antigen to said animal is taught herein.

A method of eliciting an immune response in an animal, wherein said method comprises administering an oil body-antigen complex produced by a method comprising the steps of:
a) isolating and purifying an oil body;
b) linking an antigen to said oil body to form an oil body-antigen complex; and
c) administering said oil body-antigen complex to said animal is taught herein.

A method of eliciting an immune response in an animal, wherein said method comprises administering an oil body-antigen complex produced by a method comprising the steps of:
a) introducing into a cell a chimeric nucleic acid sequence comprising:
   1) a first nucleic acid sequence capable of regulating transcription in said cell operatively linked to;
   2) a second nucleic acid sequence encoding a recombinant fusion polypeptide comprising
      (i) a first nucleic acid sequence encoding a sufficient portion of an oil body protein to provide targeting to an oil body linked in reading frame to;
      (ii) a second nucleic acid sequence encoding a linker molecule operatively linked to;
   3) a third nucleic acid sequence capable of terminating transcription in said cell;
b) growing said cell under conditions to permit expression of said fusion polypeptide in a progeny cell comprising oil bodies;
c) isolating said oil bodies comprising the linker molecule;
d) linking an antigen to said oil body via said linker molecule to form an oil body-antigen complex; and
e) administering said oil body-antigen complex to said animal is also taught herein.

A method of eliciting an immune response in an animal, wherein said method comprises administering an oil body-antigen complex produced by a method comprising the steps of:
a) introducing into a cell a chimeric nucleic acid sequence comprising:
   1) a first nucleic acid sequence capable of regulating transcription in said cell operatively linked to;
   2) a second nucleic acid sequence encoding a recombinant fusion polypeptide comprising;
      (i) a first nucleic acid sequence encoding a sufficient portion of an oil body protein to provide targeting to an oil body linked in reading frame to ;
      (ii) a second nucleic acid sequence encoding an antigen operatively linked to;
   3) a third nucleic acid sequence capable of terminating transcription in said cell;
      b) growing said cell under conditions to permit expression of said antigen in a progeny cell resulting in the formation of an oil body-antigen complex;
      c) isolating said oil body-antigen complex; and
      d) administering the said plant oil body-antigen complex to said animal is taught herein.

      The present invention provides a method for the production of an oil-body antigen complex capable of eliciting an immune response in an animal comprising:
      i) isolating and purifying of native oil bodies from oilseed plants;
      ii) modifying the oil bodies to provide a means to attach the antigen;
      iii) preparing an antigen with a linker to provide a means to attach to the oil body;
      iv) modifying the linker to permit attachment to the oil body; and
      v) coupling of antigen and oil bodies wherein modification of the oil body is biotinylation of a protein expressed in the oil body and wherein said antigen is also biotinylated and said biotinylated oil body is bound to said biotinylated antigen by streptavidin or avidin.

Yet another preferred embodiment of the present invention is a vaccine formulation comprising an oil body-antigen complex

Another preferred embodiment of the present invention is a use of a vaccine formulation comprising an oil body-antigen complex for immunizing an animal against infection by a bacterial, viral or parasitic pathogen; for immunizing an animal against cancer cells; for immunizing an animal in order to modulate the immune response involved in an autoimmune reaction; and for immunizing an animal in order to modulate the immune response involved in an allergic reaction.

### BRIEF DESCRIPTION OF THE DRAWINGS

Figure 1 is a pictorial representation of a plant oil-body.
Figure 2 is a pictorial representation of an antigen coupled to an oil-body by the use of biotin and streptavidin molecules.
Figure 3 is a pictorial representation of a plant oil-body containing a recombinant oleosin protein with an antigenic determinant that is expressed on the surface of the oil-body.
Figure 4 is a pictorial representation of two oil-body preparations, a oil-body derived from a transgenic plant containing a recombinant oleosin oil-body protein gene expressing an antigen on the oil-body surface and an antigen coupled to an oil-body by the use of streptavidin and biotin.
Figure 5 is a plasmid map of the expression vector pT7BioHis.
Figure 6 is a plasmid map of the recombinant vector pSBS2004-92 M982 TbpB N-lobe.
Figure 7 is a composite figure demonstrating the expression of *Neisseria meningitidis* TbpB N-lobe as an oleosin fusion protein in electroblots stained for protein (A) or detected with anti-oleosin antibody (B).
Figure 8 is an electroblot demonstrating that the fusion protein of oleosin and *Neisseria meningitidis* TbpB N-lobe retains binding activity for human transferrin.

### DETAILED DESCRIPTION OF THE INVENTION

### I. OIL BODIES AS ADJUVANTS

### General Description

The method of the present invention provides a means for the production of vaccines comprising oil bodies and a desired antigen. An antigen is an entity capable of reacting with the products of an immune response. An antigen commonly requires additional factors in order to induce an effective immune response. An adjuvant is any material that can increase the specific humoral and/or cellular responses to antigens. A vaccine is comprised of an antigen and adjuvant and any other components required for effective administration. Plant oil-bodies have been found to offer a safe and effective alternative to common adjuvants, can be delivered by a variety of routes of administration, and can be produced inexpensively on a large scale.

In one embodiment of the present invention the vaccine is comprised of an oil body-antigen complex.
Thus the antigen can be isolated from a variety of different sources.

Antigenic determinants that induce the production of antibody (B cell determinants) are often defined not only by the amino acids involved but also by their three dimensional orientation. In contrast, determinants for T cells are largely based on the linear sequence. Protein or polypeptide sequences can be associated with oil bodies by strong specific interactions, by covalent linkage to the oil body proteins or by expression as an oleosin-fusion sequence. With fusion at either terminus of the oleosin protein, proper presentation to the immune system can be maintained.

Haptens are small functional groups that correspond to a single antigenic determinant. Haptens are unable to be immunogenic without coupling to a carrier. Short chain peptides or carbohydrates (CHO) are classified as haptens as they are unable to induce an immune response without a potent carrier and/or adjuvant. Peptides, based on antigenic determinants derived from whole proteins, are of great interest as they can be synthesized with highest purity, thus reducing biological contaminants from recombinant production. Antigenic peptide sequences can be derived by epitope mapping of a whole protein or deducing the sequence from the DNA of phages isolated from a phage display library. Peptides and CHOs can be synthesized with reactive N-terminal or C-terminal groups included for attachment of the antigen to a carrier such as biotin or fatty acyl group (lauroyl, myristal, etc.). Peptides can also be synthesized with amino acid residues able to conformationally constrain the peptide to more resemble the 3D structure of the whole antigen.

The use of haptens may be of particular importance to the use of oil-bodies as an immunizing system. Peptides are attached by strong association (streptavidin-biotin interaction). With fusion at either terminus of the oleosin protein, proper presentation to the immune system can be maintained. The peptide sequences need not be only those known to induce the production of antibodies based on known protein sequences, but also peptide mimics of CHO sequences, specific and nonspecific sequences able to induce Tₕₑₗₚₑᵣ cells and T_{cytoloxic} cells, or increase antigen uptake by antigen presenting cells; macrophages, dendritic cells and B cells. Specific examples of peptides used in these ways (respectively) include peptide-mimics of *N. meningitidis* polysaccharide capsule; specific Tₕₑₗₚₑᵣ epitopes such as the HbsAg, HIV and malaria epitopes and specific T_{cytotoxic} sequences such as the melanoma peptide presently in clinical trials; Pan-DR (all Tₕₑₗₚₑᵣ)-binding [PaDRe] sequence, Pan-HLA-A2 (all T_{cytotoxic} )-binding sequences, or non-immune stimulation via Interleukin-1 receptor stimulation (IL-1 peptide); and complement component fragments such as C5a and C3d.

Specific examples of peptide sequences for immunization include; MART and GP-100 melanoma peptide, breast cancer peptide, Hepatitis B peptide, HCV peptide, rheumatoid arthritis, CEA peptide (GI cancer), allergy vaccines based on the peptide sequences of allergens, multiple sclerosis TCR peptide, and *Pseudomonas* adhesion peptide.

The antigens may be derived from or represent molecules from infectious agents (bacteria, viruses, parasites) and may be used to generate in immune response to eliminate or reduce the effects of infection by the infectious agent. The antigen may be derived from or represent a component of a cancer cell and be used to generate an immune response to help eliminate the cancer cells. The antigen may be derived from or represent molecules that are involved directly or indirectly in an autoimmune response and may be use to modulate the immune response to reduce the undesired effects of the autoimmune disease.

### Preparation and Properties of Oil Bodies

Oil bodies consist of a triacylglyceride matrix encapsulated by a monolayer of phospholipids and oil body proteins. Oil bodies, or oil body-like organelles, are present in animal cells, plant cells, fungal cells, yeast cells (Leber, R. et al., 1994, Yeast 10: 1421-1428), bacterial cells (Pieper-Fürst et al., 1994, J. Bacteriol. 176: 4328 - 4337) and algae cells (Rossler, P.G., 1988, J. Physiol. (London) 24: 394-400). The present invention provides a method for preparing oil bodies for use as an adjuvant comprising: 1) obtaining oil bodies from a cell; 2) washing the oil bodies; and 3) formulating the washed oil bodies into an emulsion for use as an adjuvant. The consistency of the washed oil body preparation can be adjusted by varying the water content so that it is suitable for a variety of different routes of administration. Oil body preparations with relatively high water content can be used for injection or aerosol formation suitable for parenteral or mucosal administration, respectively. Oil body preparations with a low water content, have the consistency of a cream or ointment and are suitable for topical administration. The washed oil body preparation may be obtained from any cell containing oil bodies or oil body-like organelles.

In preferred embodiments of the invention the oil bodies are obtained from a plant cell which includes cells from pollens, spores, seed and vegetative plant organs in which oil bodies or oil body-like organelles are present (Huang, 1992, Ann. Rev. Plant Physiol. 43: 177-200). The oil bodies may be obtained from a plant cell by rupturing the plant cell membrane and cell wall using any method that releases the cells constituents without substantially compromising the structural integrity of the oil bodies. More preferably, the washed oil body preparation of the subject invention is prepared from plant seeds. Accordingly, the present invention further provides a method for preparing an emulsion formulation comprising:
(1) obtaining oil bodies from plant seeds by a method that comprises:
   (a) grinding plant seeds to obtain ground seeds comprising substantially intact oil bodies;
   (b) removing solids from the ground seeds; and
   (c) separating the oil body phase from the aqueous phase;
(2) washing the oil body phase to yield a washed oil body preparation; and
(3) formulating the washed oil body preparation into an emulsion for use as an adjuvant in a vaccine formulation.

In a preferred embodiment of the invention, a liquid phase is added to the seeds prior to or while grinding the seeds.

In the context of the present invention the plant oil-bodies are recovered and purified from oilseed plants by standard methods. In preferred embodiments of the invention the oil bodies are obtained from a plant cell which includes cells from pollens, spores, seed and vegetative plant organs in which oil bodies or oil body-like organelles are present (Huang, 1992, Ann. Rev. Plant Physiol. 43:177-200).

Preferred for use herein are oil bodies obtained from plant seeds selected from the group of plant species consisting of Brazil nut (*Bertholletia excelsa*); castor (*Ricinus communis*); coconut (*Cocus nucifera*); coriander (*Coriandrum sativum*); cottonseed (*Gossypium* spp.); groundnut (*Arachis hypogaea*); jojoba (*Simmondsia chinensis*); linseed/flax (*Linum usitatissimum*); maize (*Zea mays*); mustard (*Brassica* spp. and *Sinapis alba*); oil palm (*Elaeis guineeis*); olive (*Olea europaea*); rapeseed (Brassica spp.); safflower (*Carthamus tinctorius*); soybean (*Glycine max*); squash (*Cucurbita maxima*); sunflower (*Helianthus annuus*); and mixtures thereof

Most preferred for use herein are oil bodies prepared from oilseeds such as oilseed rape or canola and related species (*Brassica napus, Brassica rapa* and other *Brassicas* and related species), flax (*Linum usitatisimum*), soybean (*Glycine max*), safflower (*Carthamus tinctorius*) or *Arabidopsis thaliana.*

Plants are grown and allowed to set seed using agricultural cultivation practices well known to a person skilled in the art. After harvesting the seed, and if desired removal of material such as stones or seed hulls (dehulling), by for example sieving or rinsing, and optionally drying of the seed, seeds are subsequently processed by mechanical pressing, grinding or crushing. In a preferred embodiment, a liquid phase is added prior to or while grinding the seeds. This is known as wet milling. Preferably the liquid is water although organic solvents such as ethanol may also be used. Wet milling in oil extraction processes has been reported for seeds from a variety of plant species including: mustard (Aguilar et al.1990, Journal of Texture studies 22:59-84), soybean (US Patent 3,971,856; Carter et al., 1974, J. Am. Oil Chem. Soc. 51:137-141), peanut (US Patent 4,025,658; US Patent 4,362,759), cottonseed (Lawhon et al.,1977, J. Am. Oil, Chem. Soc. 63:533-534) and coconut (Kumar et al., 1995, INFORM 6 (11):1217-1240). It may also be advantageous to imbibe the seeds for a time period from about fifteen minutes to about two days in a liquid phase prior grinding. Imbibing may soften the cell walls and facilitate the grinding process. Imbibition for longer time periods may mimic the germination process and result in certain advantageous alterations in the composition of the seed constituents. Preferably the added liquid phase is water.

The seeds are preferably ground using a colloid mill, such as the MZ130 (Fryma Inc.). Besides colloid mills, other milling and grinding equipment capable of processing industrial scale quantities of seed may also be employed in the here described invention including: flaking rolls, disk mills, colloid mills, pin mills, orbital mills, IKA mills and industrial scale homogenizers. The selection of the mill may depend on the seed throughput requirements as well as on the source of the seed that is employed. It is generally preferred that seed oil bodies remain substantially intact during the grinding process. Grinding of the seeds therefore results in the release of preferably less than about 50% (v/v) of the total seed oil content in the form of free oil, more preferably less than about 20% (v/v) and most preferably less than about 10% (w/w). Any operating conditions commonly employed in oil seed processing, which tend to disrupt oil bodies are unsuitable for use in the process of the subject invention. Milling temperatures are preferably between 10°C and 90°C and more preferably between 26°C and 30°C, while the pH is preferably maintained between 2.0 and 10.

Solid contaminants, such as seed hulls, fibrous material, undissolved carbohydrates and proteins and other insoluble contaminants, are removed from the crushed seed fraction. Separation of solid contaminants, may be accomplished using a decantation centrifuge, such as a HASCO 200 2-phase decantation centrifuge or a NX310B (Alpha Laval). Depending on the seed throughput requirements, the capacity of the decantation centrifuge may be varied by using other models of decantation centrifuges, such as 3-phase decanters. Operating conditions vary depending on the particular centrifuge which is employed and must be adjusted so that insoluble contaminating materials sediment and remain sedimented upon decantation. A partial separation of the oil body phase and liquid phase may be observed under these conditions.

Following the removal of insoluble contaminants, the oil body phase is separated from the aqueous phase. In a preferred embodiment of the invention a tubular bowl centrifuge is employed. In other embodiments, hydrocyclones, disc stack centrifuges, or settling of phases under natural gravitation or any other gravity based separation method may be employed. It is also possible to separate the oil body fraction from the aqueous phase employing size exclusion methods, such as membrane ultrafiltration and crossflow microfiltration. In preferred embodiments the tubular bowl centrifuge is a Sharples model AS-16 (Alpha Laval) or an AS-46 Sharples (Alpha Laval). A critical parameter is the size of the ring dam used to operate the centrifuge. Ring dams are removable rings with a central circular opening varying, in the case of the AS-16, from 28 to 36 mm and regulate the separation of the aqueous phase from the oil body phase thus governing the purity of the oil body fraction that is obtained. In preferred embodiments, a ring dam size of 29 or 30 mm is employed when using the AS-16. The exact ring dam size employed depends on the type of oil seed that is used as well as on the desired final consistency of the oil body preparation. The efficiency of separation is further affected by the flow rate. Where the AS-16 is used flow rates are typically between 750-1000 ml/min (ring dam size 29) or between 400-600 ml/min (ring dam size 30) and temperatures are preferably maintained between 26°C and 30°C. Depending on the model centrifuge used, flow rates and ring dam sizes must be adjusted so that an optimal separation of the oil body fraction from the aqueous phase is achieved. These adjustments will be readily apparent to a skilled artisan.

Separation of solids and separation of the aqueous phase from the oil body fraction may also be carried out concomitantly using a gravity based separation method such as 3-phase tubular bowl centrifuge or a decanter or a hydrocyclone or a size exclusion based separation method.

The compositions obtained at this stage in the process, generally are relatively crude and comprise numerous endogenous seed proteins, which includes glycosylated and non-glycosylated proteins and other contaminants such as starch or glucosinilates or breakdown products thereof. In accordance with the present invention the removal of a significant amount of seed contaminants is generally preferred. To accomplish removal of contaminating seed material, the oil body preparation obtained upon separation from the aqueous phase is washed at least once by resuspending the oil body fraction and centrifuging the resuspended fraction. This process yields what for the purpose of this application is referred to as a washed oil body preparation. The number of washes will generally depend on the desired purity of the oil body fraction. Depending on the washing conditions that are employed, an essentially pure oil body preparation may be obtained. In such a preparation the only proteins present would be oil body proteins. In order to wash the oil body fraction, tubular bowl centrifuges or other centrifuges such hydrocyclones or disc stack centrifuges may be used. Washing of oil bodies may be performed using water, buffer systems, for example, sodium chloride in concentrations between 0.01 M and at least 2 M, 0.1 M sodium carbonate at high pH (11-12), low salt buffer, such as 50 mM Tris-HCl pH 7.5, organic solvents, detergents or any other liquid phase. In preferred embodiments the washes are performed at high pH (11-12). The liquid phase used for washing as well as the washing conditions, such as the pH and temperature, may be varied depending on the type of seed that is used. Washing at a number of different pH's between pH 2 and pH 11-12 may be beneficial as this will allow the step-wise removal of contaminants, in particular proteins. Preferably washing conditions are selected such that the washed oil body preparation comprises less than about 75%(w/w) of all endogenously present non-oil body seed proteins, more preferably less than about 50% (w/w) of endogenously present non-oil body seed proteins and most preferably less than about 10% (w/w) of endogenously present non-oil body proteins. Washing conditions are selected such that the washing step results in the removal of a significant amount of contaminants without compromising the structural integrity of the oil bodies. In embodiments where more than one washing step is carried out, washing conditions may vary for different washing steps. SDS gel electrophoresis or other analytical techniques may conveniently be used to monitor the removal of endogenous seed proteins and other contaminants upon washing of the oil bodies. It is not necessary to remove all of the aqueous phase between washing steps and the final washed oil body preparation may be suspended in water, a buffer system, for example, 50 mM Tris-HCl pH 7.5, or any other liquid phase and if so desired the pH may be adjusted to any pH between pH 2 and pH 10.

The process to manufacture the oil body preparation may be performed in batch operations or in a continuous flow process. Particularly when tubular bowl centrifuges are used, a system of pumps operating between steps (a) and (b), (b) and (c), and (c) and (d) a continuous flow throughout the processing system is generated. In a preferred embodiment, the pumps are 1-inch M2 Wilden air operated double diaphragm pumps. In other embodiments, pumps, such as hydraulic or peristaltic pumps may be employed. In order to maintain a supply of homogenous consistency to the decantation centrifuge and to the tubular bowl centrifuge, homogenizers, such as an IKA homogenizer may be added between the separation steps. In-line homogenizers may also be added in between various centrifuges or size exclusion based separation equipment employed to wash the oil body preparations. Ring dam sizes, buffer compositions, temperature and pH may differ in each washing step from the ring dam size employed in the first separation step.

In embodiments of the invention where the oil bodies are isolated from softer tissues, for example the mesocarp tissue of olives, the techniques applied to break open the cell may vary somewhat from those used to break harder seeds. For example, pressure-based techniques may be preferred over crushing techniques. The methodology to isolate oil bodies on a small scale has been reported for isolation of oil bodies from mesocarp tissues in olive (*Olea europaea*) and avocado (*Persea americana*) (Ross et al., Plant Science, 1993, 93: 203-210) and from microspore-derived embryos of rapeseed (*Brassica napus*) (Holbrook et al., Plant Physiol.,1991, 97: 1051-1058).

The chemical and physical properties of the oil fraction may be varied in at least two ways. Firstly, different plant species contain oil bodies with different oil compositions. For example, coconut is rich in lauric oils (C₁₂), while erucic acid oils (C₂₂) are abundantly present in some *Brassica* spp. Secondly, the relative amounts of oils may be modified within a particular plant species by applying breeding and genetic engineering techniques known to the skilled artisan. Both of these techniques aim at altering the relative activities of enzymes controlling the metabolic pathways involved in oil synthesis. Through the application of these techniques, seeds with a sophisticated set of different oils are obtainable. For example, breeding efforts have resulted in the development of a rapeseed with a low erucic acid content (Canola) (Bestor, T. H.,1994, Dev. Genet. 15: 458) and plant lines with oils with alterations in the position and number of double bonds, variation in fatty acid chain length and the introduction of desirable functional groups have been generated through genetic engineering (Töpfer et al.,1995, Science, 268: 681-685). Using similar approaches a person skilled in the art will be able to further expand on the presently available sources of oil bodies. Variant oil compositions will result in variant physical and chemical properties of the oil bodies. Thus by selecting oilseeds or mixtures thereof from different species or plant lines as a source for oil bodies, or by mixing oil bodies obtained from various species or plant lines, a broad repertoire of emulsions with different textures, different properties that are beneficial to the skin and different viscosities may be acquired. Oil-bodies are typically prevalent in many types of seeds and may be conveniently isolated from oilseeds such as oilseed rape or canola and related species (*Brassica napus, Brassica rape* and other *Brassicas* and related species), flax (*Linum usitatisimum*), soybean (*Glycine max*), safflower, (*Carthamus tinctorius*) or *Arabidopsis thaliana*

### Coupling Antigen to Oil Bodies

In one embodiment of the present invention the vaccine is comprised of an oil body-antigen complex in which the antigen is attached to the oil body surface.

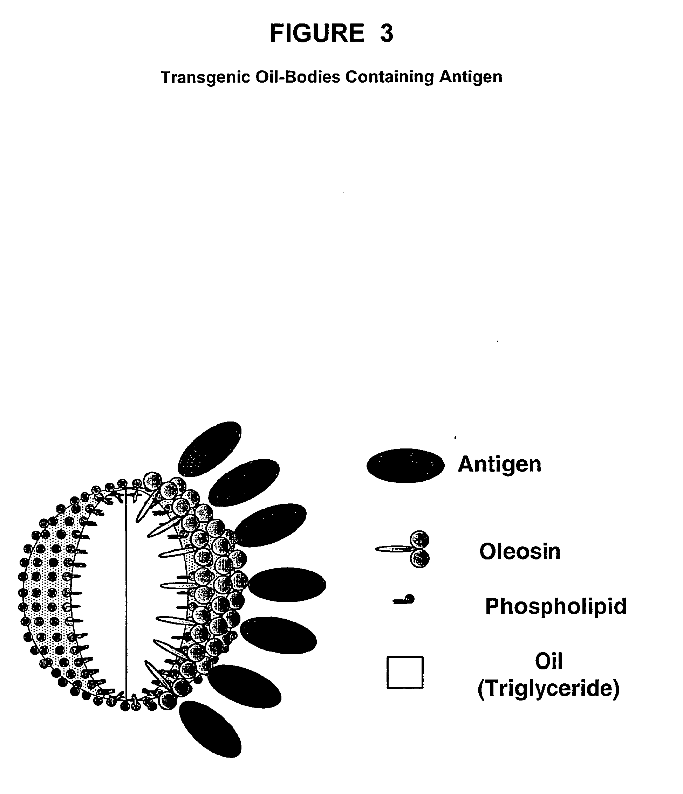

In another embodiment of the invention, the method for linking antigen to oil body includes:
1. Isolation and purification of native oil bodies from oilseed plants;
2. Modification of the oil bodies to provide a means to attach the antigen;
3. Preparation of an antigen with a linker to provide a means to attach to the oil body;
4. Modification of the linker to permit attachment to the oil body;
5. Coupling of antigen and oil bodies; wherein the oil bodies are modified to introduce biotin groups at the surface, the antigen is produced as a fusion protein with a peptide region that is enzymatically modified to introduce a biotin group, and coupling of the antigen to the oil body is accomplished by adding the multi-valent biotin-binding protein, streptavidin. Figure 3 provides an illustration of this embodiment.

Methods and compositions are provided for the production of a vaccine composition comprising isolated oil-bodies wherein one or more oil-body proteins are recombinant proteins comprising a protein sequence capable of binding or coupling to a specific antigenic determinant as taught herein. The method includes:
1. Construction of a plant transformation vector comprised of DNA sequences required to effect plant transformation containing a recombinant chimeric oleosin gene comprising an oleosin gene and a polypeptide sequence capable of binding or coupling to an antigenic determinant;
2. Transformation and recovery of recombinant plants expressing said chimeric oleosin genes;
3. Growth of said recombinant plants and recovery of seed containing recombinant oil-bodies;
4. Isolation and purification of recombinant oil-bodies comprising chimeric oleosins;
5. Coupling of an antigenic determinant to said oil-bodies

In the specific embodiment described above, the protein sequence capable of binding an antigenic determinant can be a sequence capable of binding a class of antigenic determinants; for example, a sequence capable of binding a conserved region of an antibody, or a protein capable of binding a surface carbohydrate of a microbial pathogen (a lectin). The protein sequence capable of binding an antigenic determinant can also be a protein sequence capable of binding a specific chemical moiety, such as binding of biotin by expression of an avidin or streptavidin protein sequence. This protein would be used to bind an antigenic determinant modified to contain a biotin molecule. Alternatively, the sequence could be readily modified by enzymatic means to couple to a modified antigen or coupling protein. For example, a biotinylation consensus sequence could be enzymatically biotinylated and coupled to streptavidin or a streptavidin-antigen complex. Another example would include a glycosylation site from a protein that could be enzymatically glycosylated and coupled to a lectin. Other protein sequences capable of binding a specific antigen may also be used, for example expression of an antigen-binding region derived from an antibody that is capable of selectively binding a specific antigen. The protein sequence capable of coupling to an antigenic determinant can also involve one that can generate a specific chemical moiety that will covalently couple to antigens containing another chemical moiety.

The production of the oil-body antigen complex using recombinant DNA method is taught herein. These methods include the recombinant production of antigen capable of being coupled to native oil-bodies through chemical or other means, the production of oil-bodies comprising a recombinant antigen wherein said antigen is produced as a fusion protein to one or more oil-body proteins, or production of oil-bodies comprising a recombinant protein capable of binding one or more antigens. It is readily apparent that a combination of methods may be employed to produce a vaccine composition comprising an antigen coupled to an oil-body.

Methodologies to introduce recombinant expression vectors into a plant cell also referred to herein as "transformation" are well known to the art and vary depending on the plant cell type that is selected. General techniques to transfer the recombinant expression vectors into the plant cell include electroporation; chemically mediated techniques, for example CaCl2 mediated nucleic acid uptake; particle bombardment (biolistics); the use of naturally infective nucleic acid sequences for example virally derived nucleic acid sequences or *Agrobacterium* or *Rhizobium* derived nucleic acid sequences; PEG mediated nucleic add uptake, microinjection, and the use of silicone carbide whiskers (Kaeppler et al. (1990) Plant Cell Rep. 9:415-418) all of which may be used in accordance with the present invention.

Introduction of the recombinant expression vector into the cell may result in integration of its whole or partial uptake into host cell genome including the chromosomal DNA or the plastid genome. Alternatively the recombinant expression vector may not be integrated into the genome and replicate independently of the host cell's genomic DNA. Genomic integration of the nucleic acid sequence is generally preferred as it will allow for stable inheritance of the introduced nucleic acid sequences by subsequent generations of cells and the creation of cell, plant or animal lines.

Preferred embodiments of the present invention involve the use of plant cells. Preferred plant cells used in accordance with the present invention include cells obtainable from Brazil nut (*Betholletia excelsa*); castor (*Riccinus communis*); coconut (*Cocus nucifera*); coriander (*Coriandrum sativum*); cotton (*Gossypium* spp.); groundnut (*Arachis hypogaea*); jojoba (*Simmondsia chinensis*); linseed/flax (*Linum usitatissimum*); maize (*Zea mays*); mustard (*Brassica* spp. and *Sinapis alba*); oil palm (*Elaeis guineeis*); olive (*Olea europaea*); rapeseed (*Brassica* spp.); safflower (*Carthamus tinctorius*); soybean (*Glycine max*); squash (*Cucurbita maxima*); barley (*Hordeum vulgare*); wheat (*Traeticum aestivum*) and sunflower (*Helianthus annuus*)*.*

Transformation methodologies for dicotelydenous plant species are well known. Generally *Agrobacterium* mediated transformation is preferred because of its high efficiency as well as the general susceptibility by many, if not all dicotelydenous plant species. *Agrobacterium* transformation generally involves the transfer of a binary vector (e.g. pBIN19) comprising the DNA of interest to an appropriate *Agrobacterium* strain (e.g. CIB542) by for example tri-parental mating with an *E*. *coli* strain carrying the recombinant binary vector and an *E. coli* strain carrying a helper plasmid capable of mobilization of the binary vector to the target *Agrobacterium* strain, or by DNA transformation of the *Agrobacterium* strain (Hofgen et al. Nucl. Acids. Res. 1988. 16: 9877. Other transformation methodologies that may be used to transform dicotelydenous plant species include biolistics (Sanford 1988. Trends in Biotech. 6: 299-302); electroporation (Fromm et al. 1985. Proc. Natl. Acad. Sci. USA 82: 5824-5828); PEG mediated DNA uptake (Potrykus et al. 1985. Mol. Gen. Genetics 199: 169-177); microinjection (Reich et al. Bio/Techn. 1986. 4: 1001-1004) and silicone carbide whiskers (Kaeppler et al. 1990. Plant Cell Rep. 9: 415-418). The exact transformation methodologies typically vary somewhat depending on the plant species that is used.

In a particularly preferred embodiment the oil bodies are obtained from safflower and the recombinant proteins are expressed in safflower. Safflower transformation has been described by Baker and Dyer (Plant Cell Rep. 1996.16: 106-110).

Monocotelydenous plant species may now also be transformed using a variety of methodologies including particle bombardment (Christou et al. 1991. Biotechn. 9: 957-962; Weeks et al. Plant Physiol. 1993. 102: 1077-1084; Kamm et al. Plant Cell 1990. 2: 603-618) PEG mediated DNA uptake (EP 0 292 435; 0 392 225) or *Agrobacterium*-mediated transformation (Goto-Fumiyuki et al.1999. Nature-Biotech. 17:282-286).

Plastid transformation is described in US Patents 5,451,513; 5,545,817 and 5,545,818; and PCT Patent Applications 95/16783; 98/11235 and 00/39313) Basic chloroplast transformation involves the introduction of cloned plastid DNA flanking a selectable marker together with the nucleic acid sequence of interest into a suitable target tissue using for example biolistics or protoplast transformation. Selectable markers that may be used include for example the bacterial *aadA* gene (Svab et al. 1993. Proc. Natl. Acad. Sci. USA 90: 913-917). Plastid promoters that may be used include for example the tobacco clpP gene promoter (PCT Patent Application 97/06250).

Plants may be regenerated into mature plants using plant tissue culture techniques generally known to the skilled artisan. Seeds may be harvested from mature transformed plants and used to propagate the plant line. Plants may also be crossed and in this manner it is possible in accordance with the present invention to breed lines that vary in genetic background. It is also possible to cross a plant line comprising the redox fusion polypeptide with a plant line comprising an oil body targeting protein.

Methods and compositions are taught herein for the production of a vaccine composition comprising isolated oil bodies wherein one or more oil-body proteins are recombinant proteins comprising an oleosin and an antigenic determinant. The method includes:
1. Construction of a plant transformation vector comprised of DNA sequences required to effect plant transformation containing a recombinant chimeric oleosin gene comprising an oleosin gene and a gene encoding an antigenic determinant;
2. Transformation and recovery of recombinant plants expressing said chimeric oleosin genes;
3. Growth of said recombinant plants and recovery of seed containing recombinant oil-bodies;
4. Isolation and purification of recombinant oil-bodies comprising chimeric oleosins;

In this specific embodiment, the antigen is produced as part of a recombinant oil-body. The recombinant protein may involve a fusion with oleosin or with other oil body proteins that have similar properties related to targeting to the oil body surface. Said recombinant oil-bodies can be used for immunization without further modification, or may be modified to provide for a greater immune response.

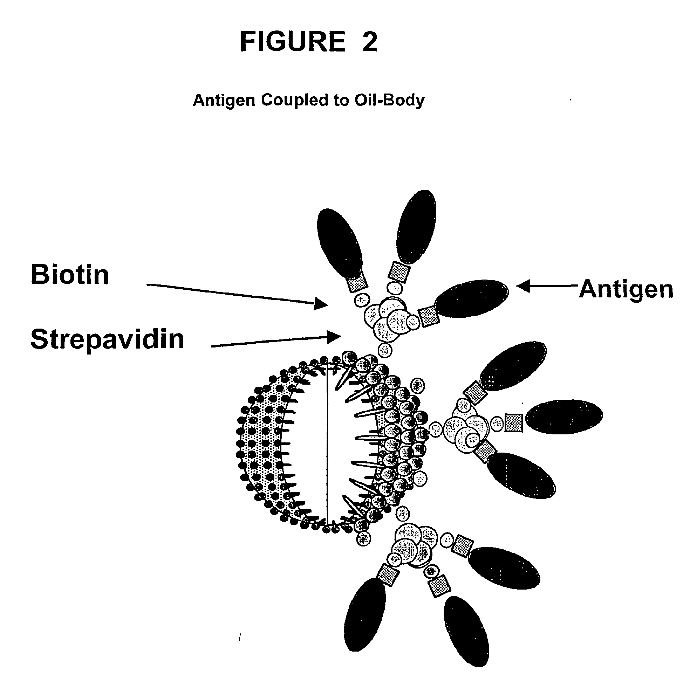

In a preferred embodiment of the invention methods and compositions are provided for the production of a vaccine composed of recombinant oil bodies comprising recombinant oleosin proteins combined with an antigenic moiety: Figure 2 provides an illustration of this embodiment.

### Formulating a Vaccine

The invention contemplates a variety of means to derive an oil body-antigen complex useful for immunization. The vaccine compositions produced using plant oil bodies can comprise one or more antigens. The vaccine compositions can also comprise one or more immunostimulatory molecules. The immunostimulatory molecules can include molecules capable of targeting the oil body to particular cells that will enhance the induction of an immune response. One example is a targeting molecule that binds to specific receptors on antigen processing cells such as dendritic cells. One specific example of an immunostimulatory molecule is cholera toxin beta subunit that is known to enhance the immune response to antigens administered to a mucosal surface.

The oil body-antigen preparation can be adjusted for water content to achieve a consistency suitable for the desired route of administration. Thus relatively dilute preparations (high water content) would be suitable for formation of aerosols for administration to the nasal or pulmonary mucosa. Intermediate preparations would be suitable for parenteral or oral administration. Concentrated (low water content) preparations have a consistency of a cream or ointment and would be suitable for topical applications. Similarly, the choice of immunostimulatory molecules could be adjusted for the desired route of administration to optimize the induction of the desired immune response.

In a preferred aspect of the present invention recombinant oil-bodies are prepared from the crushed seed of transformed Canola (*Brassica napus*) plants. Plants may be produced that express multiple different antigens by multiple transformation events or by sexual crossing of plants expressing different recombinant oleosin genes. T

Transformation vectors for *Agrobacterium* mediated transformation are prepared as disarmed binary vectors as described in US 4,940,838, US 5,464,763, "Process for the incorporation of foreign DNA into the genome of dicotyledonous plants" by R. Schilperoort *et al.* Primary transformants heterozygous for the incorporated foreign genes may be made homozygous for the trait in question by established means such as selection after selfing or via doubled haploids derived from anther or microspore culture of the primary transformants,

Multiple recombinant genes may be introduced simultaneously in the same transformation vector or successively by multiple transformation events. Plants with multiple antigenic determinants may also be formed through sexual crossing of individual plants expressing different antigens.

In another preferred embodiment of the invention the oleosins present on the surface of oil bodies linked to an antigenic determinant. A preferred method of linking the antigen to the oil body includes the steps of:
1. Biotinylation of oil-body proteins;
2. Biotinylation of purified recombinant antigen;
3. Coupling of biotinylated oil-bodies and antigen in the presence of streptavidin.

Figure 3 provides an illustration of this embodiment. In a preferred aspect of the present invention oil-bodies are prepared from the crushed seed of Canola (*Brassica napus*) and the chemical attachment of antigenic moieties produced by recombinant *E. coli* bacteria is effected by the biotin/streptavidin coupling reaction.

In a preferred embodiment of the invention the antigen is produced by bacteria such as *E. coli* strain BL21 containing recombinant DNA encoding the antigen. The recombinant DNA is constructed to provide for convenient isolation and purification of the antigen when produced (e.g. by addition of sequences allowing metal chelate or affinity chromatography).

The scope of the invention is not limited by the source or the nature of the antigen provided that it may be isolated, purified and attached to the oil-body oleosin proteins. Antigens may be produced by different *E*. *coli* strains, different bacteria, other microorganisms such as yeast, or any other protein production platform including animals, plants, fungi and cell cultures, or by non-biological means such as chemical synthesis as will be readily appreciated by those skilled in the art.

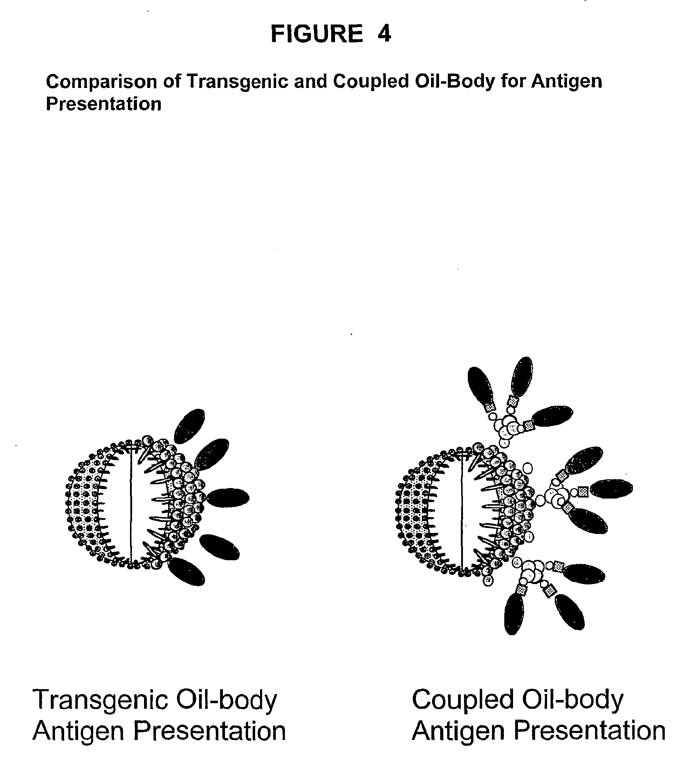

In the case of the expression of an antigen as a recombinant protein on the surface of the oil body and the coupling of an antigen to the oil body surface, a similar presentation of antigen is achieved. This is illustrated in figure 4.

Vaccines prepared using oil bodies comprising chemically attached antigenic determinants or vaccines prepared using recombinant oil bodies as described above may be administered in any appropriate way including; by injection, by presentation to a mucosal surface or by application to the skin. The present invention is directed to the production of vaccines comprising plant oil bodies that function as a platform for the presentation and delivery of antigenic substances.

The scope of the invention is not limited to the addition of a single antigen type as the parameters of the attachment process may be manipulated to introduce multiple antigens to the target oil bodies.

The scope of the invention is not limited to combinations of oil bodies and antigens as the adjuvant effect of oil body preparation could be modified by a variety of different approaches. The oil body preparations could be modified by inclusion of genetic fusions of oleosins or other oil body proteins to targeting proteins or immunomodulating molecules. Modification of the oil body preparations may include chemical or enzymatic modifications, for example glycosylation, or can include the addition of lipophilic immunostimulatory molecules. Since there are a variety of relatively small, hydrophobic molecules with immunostimulatory properties (i.e. saponins, Quil A), they could be incorporated into oil-body preparations and partition in the hydrophobic triglyceride core.

The broad method of the present invention provides the means to produce a vaccine formulation comprising an oil-body and antigen. The oil-body provides a safe adjuvant function and may eliminate the need for added adjuvants.

### II. VACCINE FORMULATIONS

The oil body formulations for use as an adjuvant in a vaccine may be formulated in a wide range of vaccine formulations. Accordingly, the present invention provides a vaccine formulation comprising oil bodies and at least one antigen.

The present invention also includes the preparation of a vaccine formulation comprising oil bodies and an antigen.

A wide variety of antigens may be formulated with the washed oil bodies of the present invention. The amount of antigen formulated will depend on the desired effect and the antigen that is selected. In general, the amount of antigen (based on transgenic antigen/oleosin fusion) varies from about 0.0001% to about 50%. More preferably however the amount of antigen in the final composition will vary from about 0.01 % to about 20% and most preferably from about 0.1% to about 10%. The antigens may be formulated into the washed oil body formulation in any desired manner (e.g. mixed, stirred) under any desired condition (e.g. heated; under pressure) and in any desired form (e.g. a liquid, solid, gel, crystal, suspension). Depending on the chemical nature of the active and the formulation methodology, the antigen may become incorporated in the final formulation in a variety of ways, for example the antigen may remain suspended in solution, or form a suspension in which the oil bodies are dispersed, or the antigen ingredients may penetrate the phospholid mono layer surrounding the oil body or the triacylglyceride matrix of the oil body.

In a preferred embodiment, the antigen is associated with the oil bodies by crosslinkers namely biotin-streptavidin and biotin-avidin crosslinkers (available from Pierce). By linking the antigen to streptavidin or avidin and biotinylating the oil bodies, or visa versa, biotinylating the antigen and linking avidin or streptavidin to the oil bodies, the antigen is crosslinked to the oil bodies via two inter-linked molecules. In a preferred embodiment, the oil bodies and antigen are biotinylated and are associated with each other by adding streptavidin. This embodiment is shown schematically in Figure 2.

A method for preparing a vaccine formulation comprising oil bodies and an antigen, said method comprising:
(a) producing an antigen in a cell;
(b) associating said antigen with oil bodies through an oil body targeting protein capable of associating with said antigen and said oil bodies;
(c) obtaining the oil bodies associated with the antigen;
(d) washing the oil bodies to obtaining washed oil body preparation comprising the antigen; and
(e) formulating the washed oil bodies associated with the antigen into a vaccine formulation is taught herein.

The term "oil body targeting protein" as used herein refers to any protein, protein fragment or peptide capable of associating with an oil body. The oil body targeting protein that is used is also capable of associating with the antigen. The term "capable of associating with the antigen" as used herein refers to covalent interactions (i.e. protein fusions) as well as non-covalent interactions between the oil body targeting protein and the antigen. The oil body targeting protein may be any oil body targeting protein, protein fragment or peptide capable of association with the antigen polypeptide and the oil bodies. The nucleic acid sequence encoding the oil body targeting peptide may be synthesized or obtained from any biological source.

Still further oil body targeting proteins may be one or more inter-linking antibodies. Particularly useful in this regard are antibodies with an affinity to oleosins. Combined inter-linked antibody-avidin-biotin or antibody-streptavidin-biotin cross-linkers may also be used in accordance with the present invention. In one embodiment the oil body targeting protein is an immunoglobulin or an immunoglobulin derived molecule, for example a bispecific single chain antibody. The generation of single chain antibodies and bi-specific single chain antibodies is known to the art (US Patents US 5,763,733, US 5,767,260 and US 5,260,203). Nucleic acid sequences encoding single chain antibodies functioning as oil body targeting proteins may be prepared from hybridoma cell lines expressing monoclonal antibodies raised against an oleosin as described by Alting-Mees *et al*. 2000. IBC's Annual International Conference on Antibody Engineering, Poster #1. In order to attain specificity for the antigen polypeptide a nucleic acid sequence encoding a second single chain antibody prepared from a monoclonal raised against the antigen polypeptide may be prepared and linked to the anti-oleosin single chain antibody. In this embodiment the oil body associates with the antigen polypeptide through non-covalent interactions of the oil body targeting protein with the antigen polypeptide and the oil body. Alternatively, the antigen polypeptide may be prepared as a fusion protein with an oil body targeting protein. For example a nucleic acid sequence encoding a single chain antibody raised against an oleosin may be fused to a nucleic acid sequence encoding antigen polypeptide

Non-immunoglobulin-based oil body targeting proteins capable of association with an antigen polypeptide may be discovered and prepared using for example phage display techniques (Pharmacia Biotech Catalogue Number 27-9401-011 Recombinant Phage Antibody System Expression Kit).

Oil body targeting proteins may also be chemically modified. For example oleosins may be modified by changing chemical modification of the lysine residues using chemical agents such as biotinyl-N-hyrdoxysuccinimide ester resulting a process referred to as biotinylation. Conveniently this is accomplished by in vitro biotinylation of the oil bodies. In vivo biotinylation may be accomplished using the biotinylation domain peptide from the biotin carboxy carrier protein of E. *coli* acetyl-CoA carboxylase (Smith et al. 1998. Nucl. Acids. Res. 26: 1414-1420). Avidin or streptavidin may subsequently be used to accomplish association of the antigen with the oil body.

In a preferred embodiment the oil body targeting protein is an oil body protein such as for example an oleosin or a sufficient portion derived thereof capable of targeting to an oil body. Nucleic acid sequences encoding oleosins are known to the art. These include for example the Arabidopsis oleosin (Van Rooijen et al. 1991. Plant Mol. Bio. 18:1177-1179); the maize oleosin (Qu and Huang. 1990. J. Biol. Chem. Vol. 265 4:2238-2243); rapeseed oleosin (Lee and Huang. 1991. Plant Physiol. 96:1395-1397); and the carrot oleosin (Hatzopoulos et al. 1990. Plant Cell Vol. 2, 457-467.). The antigen polypeptide may be fused to the oil body protein. The methodology is further described in US patent 5,650,554. In such an embodiment the oil bodies and the associated antigen polypeptide can conveniently be isolated in one step. The antigen polypeptide may be fused to the N-terminus as well as to the C-terminus of the oil body protein (as described in: van Rooijen and Moloney 1995. Plant Physiol. 109:1353-1361) and fragments of the oil body protein such as for example the central domain of an oleosin molecule, or modified versions of the oil body protein may be used. This embodiment is shown schematically in Figure 3.

New oil body proteins may be discovered for example by preparing oil bodies (described in further detail below) and identifying proteins in these preparations using for example SDS gel electrophoresis. Polyclonal antibodies may be raised against these proteins and used to screen cDNA libraries in order to identify nucleic acid sequences encoding oil body proteins. The methodologies are familiar to the skilled artisan (Huynh et al. 1985. in DNA Cloning Vol. 1. a Practical Approach ed. DM Glover, IRL Press, pp 49-78). New oil body proteins may further be discovered using known nucleic acid sequences encoding oil body proteins (e.g. the *Arabidopsis,* rapeseed, carrot and corn nucleic acid sequences) to probe for example cDNA and genomic libraries for the presence of nucleic acid sequences encoding oil body proteins.

Accordingly, a method for the preparation of a vaccine formulation comprising:
(a) introducing into a cell a chimeric nucleic acid sequence comprising:
   1) a first nucleic acid sequence capable of regulating transcription in said cell operatively linked to;
   2) a second nucleic acid sequence encoding a recombinant fusion polypeptide comprising (i) a first nucleic acid sequence encoding a sufficient portion of an oil body protein to provide targeting to an oil body linked in reading frame to (ii) a second nucleic acid sequence encoding an antigen operatively linked to;
   3) a third nucleic acid sequence capable of terminating transcription in said cell;
(b) growing said cell under conditions to permit expression of said antigen in a progeny cell comprising oil bodies;
(c) isolating said oil bodies from comprising the antigen;
(d) washing said oil bodies to obtain a washed oil body preparation comprising the antigen; and
(e) formulating said oil bodies comprising the antigen into a vaccine formulation is taught herein.

One skilled in the art will appreciate that the antigen used in the vaccines of the invention can be any antigen to which one wishes to generate an immune response. The scope of the invention is not limited by the type of antigen used or the means by which the antigen is produced. Antigens may consist of peptides, proteins, carbohydrate or synthetically produced chemicals. The antigen may be similar or identical to the natural molecule against which an immune response is desired or may simply resemble the natural molecule sufficiently to be able to induce a response against the natural molecule. Due to the wide range of possibilities for production and use of antigens it is impossible to provide a comprehensive list of potential antigens that could be included in immunizations with oil bodies and thus only examples that may be reflective of the type of antigens that could be considered are provided.

The antigens may be derived from or represent molecules from infectious agents (bacteria, viruses, parasites) and may be used to generate in immune response to eliminate or reduce the effects of infection by the infectious agent. The antigen may be derived from or represent a component of a cancer cell and be used to generate an immune response to help eliminate the cancer cells. The antigen may be derived from or represent molecules that are involved directly or indirectly in an autoimmune response and may be use to modulate the immune response to reduce the undesired effects of the autoimmune disease.

Peptides and proteins antigens can be derived from or represent different types of proteins from pathogenic organisms and be used to induce an immune response that reduces or eliminates the pathogen or the effects that the pathogen has on the host. The various types of proteins can be classified on the basis of how they are produced or alternatively on the role that the protein plays in the interaction of the pathogen with the host.

One type of protein is secreted by a bacterial or parasitic pathogen and can be subclassified on the basis of its function or role. Secreted proteins include toxins secreted by bacterial or parasitic pathogens. Examples of secreted bacterial toxins include diptheria toxin, pertussis toxin, dermnecrotic toxin, tetanus toxin, *E*. *coli* heat-labile toxin, cholera toxin, shiga toxin, *Staphylococcus* toxin, and toxic shock syndrometoxin among many others. Another example of secreted proteins that could serve as antigens include proteases such as elastase, metaloprotease , IgA protease (from *Haemophilus influenzae, Neisseria spp.* or *Streptococcus pneumoniae*) or hyaluronidase (from *Streptococcus* or *Staphylococcus*). Other examples of secreted proteins that could serve as useful antigens include haemolysins or leukotoxins including streptolysin O or S, pneumolysin or leukotoxins from *Pasteurella haemolytica, Pasteurella multocida, Actinobacillus pleuropneumonia* or *Actinobacillus actinomycetencomitans.* More examples of secreted proteins are enzymes such as kinases including streptokinase and staphylokinase. Another example of secreted proteins are those that may be secreted into the eukaryotic host cell by means of the bacterial type III secretion system and include effector proteins from many Gram-negative bacterial species including *Yersinia* (invasin), *Listeria* (internalin) and *Salmonella* (subversin).

A second type of protein is a surface molecule of a pathogenic organism. As with secreted proteins, the surface proteins can be subclassified based on the function that the protein provides for pathogen. In Gram-negative bacteria, porin proteins that are involved in the movement of small molecules across the outer membrane are being evaluated as potential vaccine antigens against infections by *Neisseria spp., Pseudomonas aeruginosa* and *Escherichia coli* among many others. A second type of surface protein are surface receptors or binding proteins that are involved in transport functions or binding to host extracellular matrix proteins. These include the transferrin and lactoferrin receptor proteins from *Neisseria spp., Haemophilus influenzae, Moraxella catarrhalis, Pasteurella haemolytica, Actinobacillus pleuropneumoniae* and many other species, heme or hemoglobin binding proteins and siderophore receptors and fibrinogen-binding protein from *Streptococcus.* A third type of surface protein is an adhesin, which is involved in attachment to or adherence to the host cells directly or via extracellular host proteins. These include components of pili or fimbria from *Neisseria, Haemophilus, Pseudomonas, Escherichia coli, Streptococcus* and many other Gram-negative and Gram-positive bacteria. They also include surface adhesins such as intimin from *E*. *coli,* M proteins from *Streptococcus* species, the high molecular weight adhesins from non-typable *Haemophilus influenzae* and Usp proteins from *Moraxella catarrhalis.* Another type of surface antigen is one involved in motility such as flagellar proteins in *Pseudomonas* and *Burkholderia* species , members of the Enterobacteriacea and many other bacterial species. There are also many surface proteins for which the function is unknown which are being evaluated as potential vaccine antigens.

Another type of surface protein is a protein found on the surface of a viral particle. This includes capsid proteins such as the polio capsid proteins, group specific antigens, and envelope proteins such as Hepatitis B surface antigen, glycoproteins and hemagglutinins.

Carbohydrates are important surface molecules of pathogenic organisms and of host cells and are important antigens for infectious diseases and cancer. Antibody responses against carbohydrates can be accomplished by immunizing with carbohydrates mixed or conjugated with other molecules or my immunizing with proteins that mimic carbohydrate antigens.

Many pathogenic organisms have surface capsules consisting of carbohydrate polymers. Bacterial capsules from *Neisseria meningitidis, Streptococcus pneumoniae, Streptococcus* groups A and B and *Haemophilus influenzae* are examples of capsules used for vaccine production and development. Purified capsular carbohydrates were used for the first generation of capsular vaccines and improved versions of these vaccines are available (*Haemophilus influenzae*) or are being tested (*Neisseria meningitidis, Streptococcus pneumoniae*). Capsular vaccines are also being considered for fungal diseases such a histoplasmosis and crytococcosis. Lipopolysaccharides and lipooligosaccharides are prominent surface components of all Gram-negative bacterial species and would be very useful targets for the immune response were it not for the intrinsic toxicity of these molecules. Glycolipids are significant components of the surface of mycobacteria (i.e. *Mycobacterium tuberculosis*) and mycoplasma and are potential vaccine antigens. Blood group antigens such as the Lewis blood group antigens (for breast cancer metastases) are important for vaccine consideration in cancer therapy.

Protein or peptide antigens may also be administered in the vaccine formulation as a nucleic acid encoding the antigen. Such nucleic acids include free or naked RNA or DNA or in a vector. In a preferred embodiment, the nucleic acid sequence is contained in a vector or plasmid. In one embodiment, the vector may be viral such as poxvirus, adenovirus or alphavirus. Preferably the viral vector is incapable of integration in recipient animal cells. The elements for expression from said vector may include a promoter suitable for expression in recipient animal cells.

The following optional ingredients and mixtures thereof represent non-limiting examples of ingredients that may be additionally formulated with oil bodies and the antigen in order to prepare a vaccine formulation.

### Carriers/Auxiliary Agents

The vaccines of the invention may be in admixture with a suitable carrier, diluent, or excipient such as sterile water, physiological saline, glucose or the like to form suitable vaccine formulations. The vaccines can also be lyophilized. The vaccines may also contain auxiliary substances such as wetting or emulsifying agents, pH buffering agents, gelling or viscosity enhancing additives, preservatives, flavoring agents, colors, and the like, depending upon the route of administration and the preparation desired. In this regard, reference can be made to U.S. Patent No. 5,843,456. Reference can also be made to the textbook: Vaccine Design: the Subunit and Adjuvant Approach. Michael F. Powell and Mark J. Newman, eds. Plenum Press, New York, 1995.

### Adjuvants

Although the oil bodies themselves act as an adjuvant in the vaccines of the invention, the vaccine may additionally include other adjuvants. A wide range of extrinsic adjuvants can provoke potent immune responses to antigens. These include saponins complexed to membrane protein antigens (immune stimulating complexes), pluronic polymers with mineral oil, killed mycobacteria and mineral oil, Freund's complete adjuvant, bacterial products such as muramyl dipeptide (MDP) and lipopolysaccharide (LPS), as well as lipid A, and liposomes.

U.S. Patent No. 4,855,283 granted to Lockhoff et al. on August 8, 1989 teaches glycolipid analogues including N-glycosylamides, N-glycosylureas and N-glycosylcarbamates, each of which is substituted in the sugar residue by an amino acid, as immunomodulators or adjuvants. Thus, Lockhoff et al. (1991. Chem. Int. Ed. Engl. 30:1611-1620) reported that N-glycolipid analogs displaying structural similarities to the naturally occurring glycolipids, such as glycophospholipids and glycoglycerolipids, are capable of eliciting strong immune responses in both herpes simplex virus vaccine and pseudorabies virus vaccine. Some glycolipids have been synthesized (from long chain-alkylamines and fatty acids that are linked directly with the sugars through the anomeric carbon atom) to mimic the functions of the naturally occurring lipid residues.

U.S. Patent No. 4,258,029 granted to Moloney teaches that octadecyl tyrosine hydrochloride (OTH) functions as an adjuvant when complexed with tetanus toxoid and formalin inactivated type I, II and III poliomyelitis virus vaccine. Nixon-George et al. (1990. J. Immunol. 14:4798-4802) have also reported that octadecyl esters of aromatic amino acids complexed with a recombinant hepatitis B surface antigen enhanced the host immune responses against hepatitis B virus.

Adjuvant compounds may also be chosen from the polymers of acrylic or methacrylic acid and the copolymers of maleic anhydride and alkenyl derivative. Adjuvant compounds are the polymers of acrylic or methacrylic acid that are cross-linked, especially with polyalkenyl ethers of sugars or polyalcohols. These compounds are known by the term carbomer (Phameuropa Vol. 8, No. 2, June 1996). Preferably, a solution of adjuvant according to the invention, especially of carbomer, is prepared in distilled water, preferably in the presence of sodium chloride, the solution obtained being at acidic pH. This stock solution is diluted by adding it to the desired quantity (for obtaining the desired final concentration), or a substantial part thereof, of water charged with NaCl, preferably physiological saline (NaCl 9 g/l) all at once in several portions with concomitant or subsequent neutralization (pH 7.3 to 7.4), preferably with NaOH. This solution at physiological pH will be used as it is for mixing with the vaccine, which may be especially stored in freeze-dried, liquid or frozen form. The polymer concentration in the final vaccine composition will be 0.01% to 2% w/v, more particularly 0.06 to 1% w/v, preferably 0.1 to 0.6% w/v.

Persons skilled in the art can also refer to U.S. Patent No. 2,909,462 which describes such acrylic polymers cross-linked with a polyhydroxylated compound having at least 3 hydroxyl groups (preferably not more than 8), the hydrogen atoms of the at least three hydroxyls being replaced by unsaturated aliphatic radicals having at least 2 carbon atoms. The preferred radicals are those containing from 2 to 4 carbon atoms (e.g. vinyls, allyls and other ethylenically unsaturated groups). The unsaturated radicals may themselves contain other substituents, such as methyl. The products sold under the name Carbopol (BF Goodrich, Ohio, USA) are particularly appropriate. They are cross-linked with allyl sucrose or with allyl pentaerythritol. Among them, there may be mentioned Carbopol (for example, 974P, 934P and 971P). Among the copolymers of maleic anhydride and alkenyl derivative, the copolymers EMA (Monsanto; which are copolymers of maleic anhydride and ethylene, linear or cross-linked, (for example cross-linked with divinyl ether)) are preferred. Reference may be made to J. Fields et al. (Nature, 1960, 186: 778-780) for a further description of these chemicals.

In one aspect of this invention, adjuvants useful in any of the embodiments of the invention described herein are as follows. Adjuvants for parenteral immunization include aluminum compounds (such as aluminum hydroxide, aluminum phosphate, and aluminum hydroxy phosphate). The antigen can be precipitated with, or adsorbed onto, the aluminum compound according to standard protocols. Other adjuvants such as RIBI (ImmunoChem, Hamilton, MT) can also be used in parenteral administration.

Adjuvants for mucosal immunization include bacterial toxins (e.g., the cholera toxin (CT), the E. coli heat-labile toxin (LT), the *Clostridium difficile* toxin A and the pertussis toxin (PT), or combinations, subunits, toxoids, or mutants thereof). For example, a purified preparation of native cholera toxin subunit B (CTB) can be of use. Fragments, homologs, derivatives, and fusion to any of these toxins are also suitable, provided that they retain adjuvant activity. Preferably, a mutant having reduced toxicity is used. Suitable mutants have been described (e.g., in WO 95/17211 (Arg-7-Lys CT mutant), WO 96/6627 (Arg-192-Gly LT mutant), and WO 95/34323 (Arg-9-Lys and Glu-129-Gly PT mutant)). Additional LT mutants that can be used in the methods and compositions of the invention include, for example Ser-63-Lys, Ala-69-Gly, Glu-110-Asp, and Glu-112-Asp mutants. Other adjuvants (such as a bacterial monophosphoryl lipid A (MPLA) of various sources (e.g., E. coli, *Salmonella minnesota, Salmonella typhimurium,* or *Shigella flexneri,* saponins, or polylactide glycolide (PLGA) microspheres) can also be used in mucosal administration.

Adjuvants useful for both mucosal and parenteral immunization include polyphosphazene (for example, WO 95/2415), DC-chol (3 b-(N-(N',N'-dimethyl aminomethane)-carbamoyl) cholesterol (for example, U.S. Patent No. 5,283,185 and WO 96/14831) and QS-21 (for example, WO 88/9336).

### Emulsion Stabilizing Agents

In a preferred embodiment of the present invention, the washed oil body preparation is stabilized so that an emulsion is obtained which may be stored for longer periods of time. For the purpose of the present application the term "stabilized oil body preparation" *refers to an oil* body emulsion that is prepared so that the oil body emulsion does not undergo undesirable physical or chemical alterations when the oil body emulsion is stored for long periods of time. Preferably the oil body preparation is prepared to be stable for at least 1 month, more preferably the preparation is stable for at least 1 year, and most preferably the preparation is stable at least 2 years when stored at room temperature. In a further preferred embodiment, the oil body emulsion is prepared so that the preparation additionally can withstand temperature fluctuations such as those which typically occur in non-temperature controlled environments for example during transport. In a stable oil body preparation alterations over time with respect to color, odor, viscosity, texture, pH and microbial growth are minimal or absent.

Generally, the emulsion formulations will be treated such that contamination by bacteria, fungi, mycoplasmas, viruses and the like or undesired chemical reactions, such as oxidative reactions are prevented. In preferred embodiments this is accomplished by the addition of preservatives, for example sodium metabisulfite; Glydant Plus; Phenonip; methylparaben; propylparaben; Germall 115; Germaben II; phytic acid; and mixtures thereof. The preparation may also be stabilized by irradiation, for example by ionizing radiation such as cobalt-60 or cesium-137 irradiation or by ultraviolet irradiation or by heat treatment for example by pasteurization in a constant temperature water bath at approximately 65°C for 20 minutes. The pasteurization temperature preferably ranges between 50°C and 90°C and the time for pasteurization preferably ranges between 15 seconds to 35 minutes.

Oxidative reactions may be prevented by the addition of anti-oxidants such as for example butylated hydroxytoluene (BHT); butylated hydroxyanisol (BHA); ascorbic acid (vitamin C); tocopherol; phytic acid; citric acid; pro-vitamin A; and mixtures thereof.

The physical stability of the formulation may be further enhanced by the addition of for example an emulsifier such as an Arlacel such as Arlacel 165 or Glucamate LT or by the addition of viscosity modifiers such as such as cetyl alcohol; glycerol or Keltrol. The emulsion may be thickened and stabilized using gelling agents such as cellulose and derivatives; Carbopol and derivatives; carob; carregeenans and derivatives; xanthane gum; sclerane gum; long chain alkanolamides; bentone and derivatives; Kaolin USP; Veegum Ultra; Green Clay; Bentonite NFBC; and mixtures thereof. These agents are typically present in concentrations less than about 2% by weight.

The oil body preparation may also be further stabilized by modifying the pH and by modifying the ionic strength for example by adjusting the concentration of calcium or sodium ions.

The following additional ingredients may be formulated with the stabilized oil body formulation. While in preferred embodiments of the present invention, the oil bodies are stabilized prior to the formulation with these additional ingredients, it is nevertheless possible to formulate the oil body preparation and stabilize the final formulation.

### III. USES OF THE VACCINE FORMULATIONS

The subject invention is directed toward the production of oil body preparations that are, useful in a wide variety of applications including as an adjuvant in a vaccine formulation.

Accordingly, the present invention provides a method of eliciting an immune response comprising administering an effective amount of a vaccine formulation comprising oil bodies and an antigen to an animal in need thereof.

The term "eliciting an immune response" is defined as causing, enhancing, or improving any response of the immune system, for example, of either a humoral or cell-mediated nature. Whether a vaccine or antigen elicits an immune response can be assessed using assays known to those skilled in the art including, but not limited to, antibody assays (for example ELISA assays), antigen specific cytotoxicity assays and the production of cytokines (for example ELISPOT assays).

The term "an effective amount" of the vaccine of the present invention is defined as an amount effective, at dosages and for periods of time necessary to achieve the desired result (e.g. elicit an immune response). The effective amount of a compound of the invention may vary according to factors such as the disease state, age, sex, and weight of the animal. Dosage regimes may be adjusted to provide the optimum therapeutic response. For example, several divided doses may be administered daily or the dose may be proportionally reduced as indicated by the exigencies of the therapeutic situation.

The term "antigen" as used herein refers to any molecule to which one wishes to elicit an immune response.

The term "vaccine" as used herein refers to any composition capable of eliciting an immune response.

The term "animal" as used herein includes all members of the animal kingdom, including humans. Preferably, the animal to be treated is a human.

The term "administering" is defined as any conventional route for administering an antigen to an animal for use in the vaccine field as is known to one skilled in the art. This may include, for example, administration via the topical, oral and parenteral (i.e. subcutaneous, intradermal, intramuscular, etc.) routes and further includes, transdermal and mucosal delivery, including mucosal delivery accomplished by oral feeding, inhaling and through the membranes accessible through the terminal portions of the large intestine.

A particularly preferred method of immunizing an animal with the vaccine encompasses a prime-boost protocol. Typically, a prime-boost protocol involves an initial administration of the vaccine followed by a boost of the vaccine. This protocol will elicit an enhanced immune response relative to the response observed following only one administration of the vaccine. An example of a prime-boost methodology/protocol is described in WO 98/58956. In the prime-boost protocol, the route of administration for the priming does not have to be the same route as used for the boosting. As described in Example 11, the prime may be administered parenterally and the boost may be administered transdermally.

The vaccine formulation may be administered with other agents including other adjuvants as well as immune stimulatory molecules including cytokines.

### Example 1: Modification of native oil-bodies for binding antigens.

In this example, the use of native oil-bodies derived from non-transgenic plants for antigen delivery is described. The isolated native oil-bodies were chemically modified to contain biotin molecules covalently linked to oil body proteins such as oleosins. These modified oil bodies are able to bind antigen-antigen complexes, thus providing a vaccine composition containing oil bodies, antigen and a streptavidin-coupling moiety. To carry out the chemical modification of oil bodies, plant seeds from the oilseed plant *Brassica napus* were used for the isolation of oil-bodies. All procedures were performed under sterile conditions. Typically, 2 - 3 grams of mature seeds were first surface sterilized by treatment with 70% ethanol for 15 min at room temperature. The seeds were washed 2 to 3 times with sterile saline, then crushed in a pre-sterilized mortar with pestle using enough sterile saline to maintain liquid consistency. The crushed seed suspension was diluted to 40 mls with saline and transferred to a 50 ml polypropylene tube and centrifuged at 3500 rpm (10,000 x g) for 30 minutes. The "fat pad" (containing the oil-bodies) was transferred to a 50 ml polypropylene containing 40 ml of sterile saline buffer and re-centrifuged. This washing step was repeated twice and then the final fat pad was re-suspended in small amount of sterile saline. Protein content was determined and the solution adjusted to a concentration of approximately 10 mgs oleosin (oil-body protein) per ml of solution. A total of 20 ml of the biotinylation reagent N-Hydroxysuccinimidobiotin (NHS-biotin), dissolved at a concentration of 12.5 mg/ml in dimethyl formamide was added per mg of oleosin. After mixing gently for 30 min to 1 hour, the biotinylated oil-bodies were centrifuged for 20 min and the undernatant removed. The biotinylated oil-bodies were resuspended in saline and recentrifuged. This wash was repeated and the fat layer resuspended in saline to a final concentration of 10 mg oleosin (oil-body protein) per ml. These modified oil-bodies are then used for coupling to antigen-antigen complexes.

### Example 2. Production of recombinant antigens

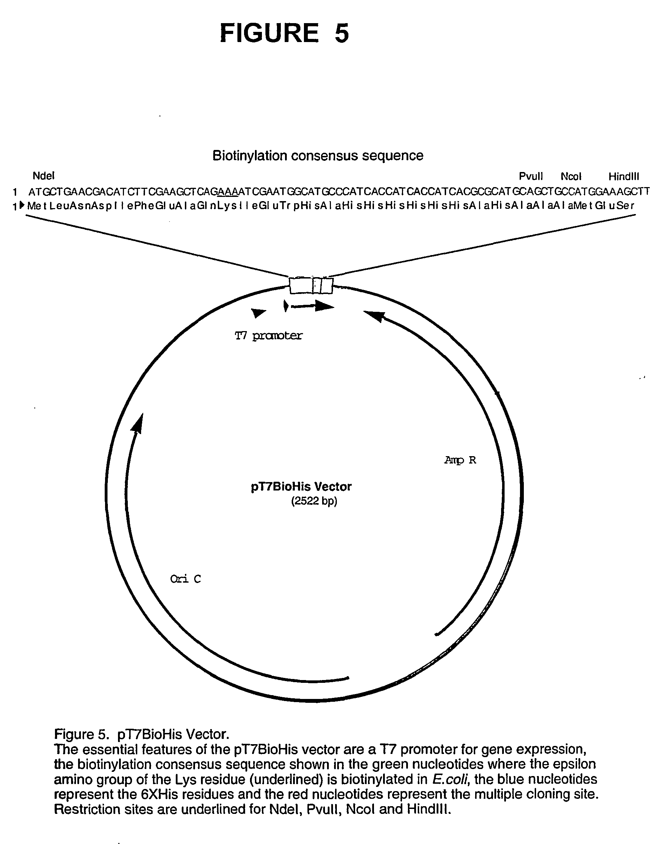

A novel expression system for production of recombinant antigen was employed to produce antigen that is easily purified and contains a single biotin moiety at a selected region. The expression vector can be induced to express in *E*. *coli* and contains a T7 promoter and a region encoding an N-terminal biotin consensus sequence (Schatz, P. J. 1993. Use of peptide libraries to map the substrate specificity of a peptide-modifying enzyme: A 13 residue consensus peptide specifies biotinylation in Escherichia coli. Bio/Technology 11:1138-1143), a polyhistidine segment and a multiple cloning site (MCS) to facilitate fusion in frame to foreign genes. The vector is referred to as pT7biohistag. The restriction map of this vector is shown in figure 5. Expression in *E*. *coli* from this vector containing a coding sequence inserted in-frame into the MCS results in a recombinant fusion protein that can readily be purified by metal-chelate chromatography due to the polyhistidine region. The recombinant protein also contains a biotin moiety attached to the lysine residue present in the N-terminal biotin consensus sequence, the biotinylation being carried out the BirA protein in the *E*. *coli* cells. It is noted that in some cases, particularly those cases where recombinant protein expression is very high in this system, the proportion of recombinant protein that is fully biotinylated may be reduced. Accordingly, in these instances, the purified protein can be fully biotinylated by the addition of biotin, ATP and a recombinant form of the BirA protein (Tsao, K. L. et al. 1996. Gene 169:59-64). Recombinant antigen is produced in *E*. *coli* strain HMS174DE3 pLysS. Bacterial cells are grown and the expression of the recombinant gene induced by 0.5 mM IPTG, which causes the expression of the pT7 biohistag vector. Following induction and growth for a period of time, cells are harvested, subjected to French press lysis, centrifuged to remove cellular debris and membranes and the antigen purified from the supernatant by nickel chelate affinity matrix chromatography. The purified antigen was fully biotinylated by incubation in the presence of a GST-BirA (glutathione-S-transferase) fusion protein with biotin and ATP added. The BirA was removed by a GST affinity chromatography column and the fully biotinylated antigen was repurified by metal chelate chromatography as above. This procedure allows for the production of recombinant antigen containing a biotin moiety.

### Example 3. Coupling of oil-bodies and antigens.

In this example, biotinylated oil-bodies and biotinylated antigens were combined in the presence of streptavidin to form an oil-body-antigen complex. One mole of streptavidin can bind four moles of biotin, thus streptavidin can be used to link or couple the biotinylated antigen to the biotinylated oil-bodies. In order to couple the biotinylated antigen to the biotinylated oil-bodies, the biotinylated antigen was premixed with streptavidin and this mixture was then added to the biotinylated oil bodies. This stepwise coupling allows control of the amount of antigen that is coupled to the oil-body surface. As a control, adding a large excess of free biotin allows for the isolation of biotinylated oil-bodies without antigen attached. All preparations of antigen, streptavidin and oil-bodies were made in sterile saline. Non-particulate preparations were filter sterilized. Biotinylated antigen was combined with streptavidin (SA) at 2.5:1 molar ratio, then the antigen-SA complex was added to biotinylated oil-bodies, and mixed vigorously. The amount of antigen-SA complex added to the biotinylated oil-body was typically adjusted to emulate a 1 - 10% expression of oleosin fusion protein in a transgenic oil-body. Thus the antigen:oleosin molar ratio was 1.25:100 or 2.5:100 in most experiments and up to 1:10 in some experiments. The coupled oil-body-antigen-SA mixture was used for immunization of test animals.

### Example 4. Production of an oil-body - antigen complex comprising a recombinant surface antigen.

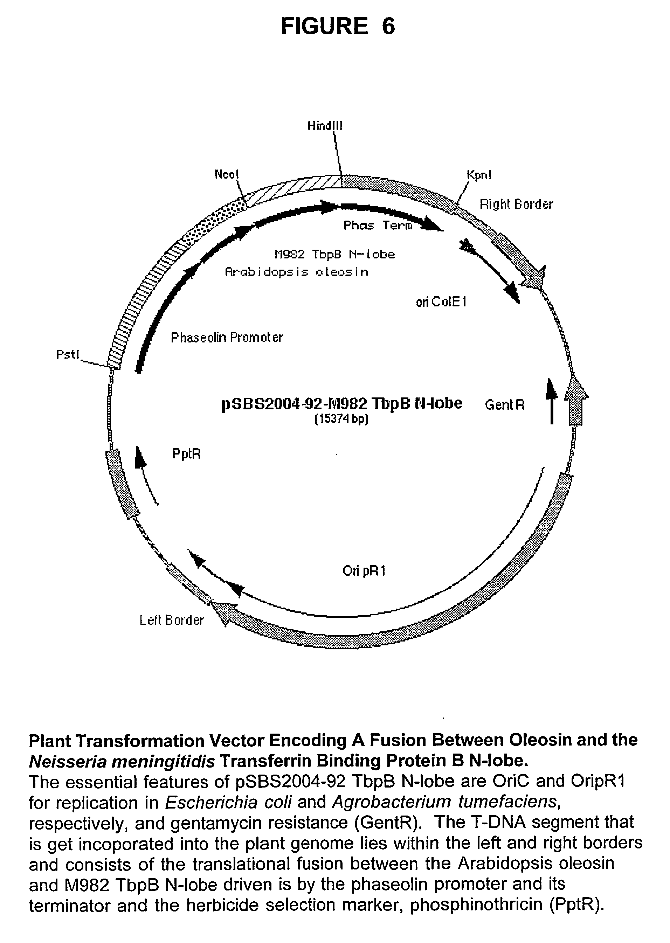

In this example, a representative surface antigen was cloned into the vector pT7biohistag and expressed. The transferrin binding protein B (TbpB) from *Neisseria meningitidis* was used as a representative surface antigen as it is a candidate for a vaccine for meningococcal meningitis (Danve, B. *et al.* 53. 1998. Eleventh International Pathogenic Neisseria Conference). The coding region of the transferrin binding protein was isolated by PCR using primers that were modified to contain convenient restriction sites for cloning into the pT7biohistag vector. The resultant vector, called pT7BioHisM982TbpB was transformed into *E*. *coli.* The sequence of the cloned gene was confirmed by DNA sequencing, the restriction map is shown in Figure 6. The *E*. *coli* strain containing the vector was grown to mid-log phase, antigen expression was induced and the recombinant antigen purified as described in example 2. The recombinant antigen was fully biotinylated as described in example 2, and coupled to biotinylated oil-bodies as described in example 3. The TbpB-oil-body-streptavidin complex was used to immunize animals.

### Example 5. Expression of an antigen as an oleosin fusion in oilseed plants.

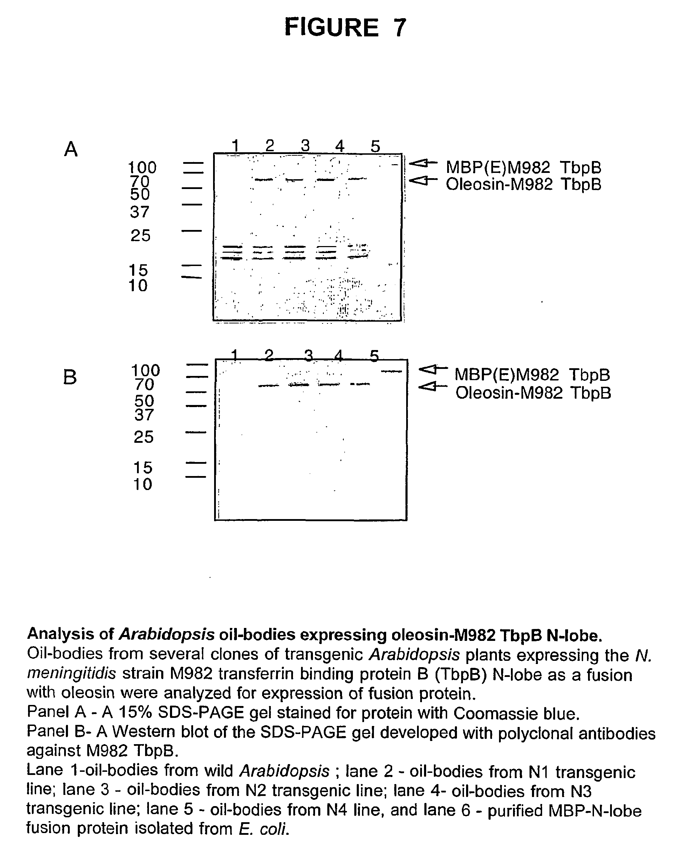

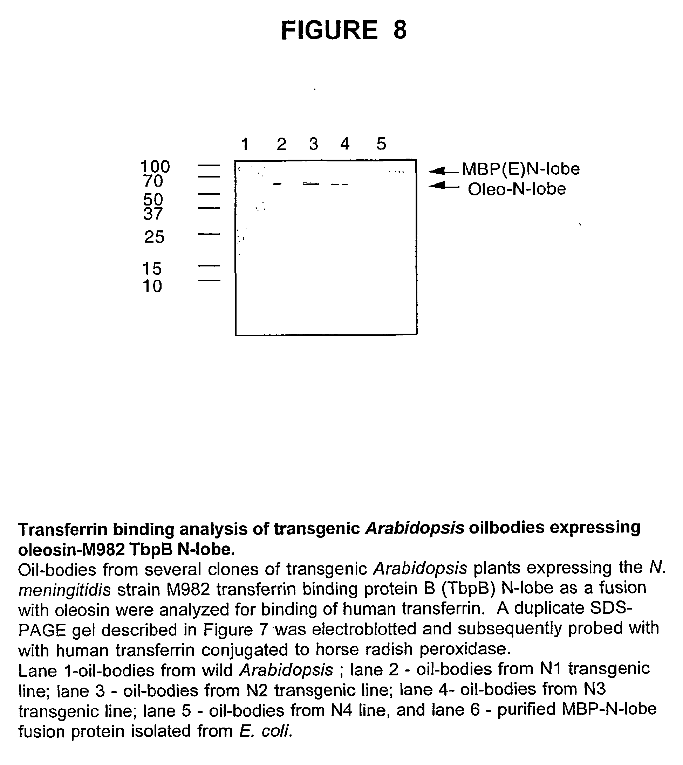

In this example, we have prepared a transgenic plant, which expresses an oleosin-M982 TbpB N-lobe fusion that associates with oil bodies. The oil seed plant used in this example is *Arabidopsis thaliana.* A translation fusion between the oleosin 18kDa and the coding region of M982 TbpB N-lobe under the control of a seed specific promoter was cloned into the binary vector pSBS2004. The resultant vector, pSBS2004-92 M982TbpB N-lobe is shown in Figure 8 was used to transform *Agrobacterium tumefaciens* strain EHA101. The transformed *Agrobacterium* strain was then used to transformed *A. thaliana* (Bechtold, N., Ellis J., and Pelletier, G. 1993. In planta Agrobacterium-mediated gene transfer by infiltration of adult plants. C.R. Acad. Sci. Paris, Life Sciences 316:1194-1199). Infected plants were allowed to mature and set seeds. Putative transgenic seeds were sown onto appropriate germination media in the presence of the herbicide, phosphinothricin (PPT). Transgenic plants that survived selection were allowed to mature and set seeds. Transgenic oil bodies expressing M982TbpB N-lobe as an oleosin fusion were isolated as described in example 1. Figure 9A shows is a Coomassie blue stained gel of transgenic oil body proteins isolated from transgenic seeds expressing M982 TbpB N-lobe as an oleosin fusion. Figure 7A shows that the oleosin-M982 TbpB fusion has an approximate molecular mass of 58.0 kDa. Figure 7B shows a western blot of the SDS gel using antibodies against M982 TbpB. The figure shows that the oleosin-M982 TbpB N-lobe fusion can be recognized by the polyclonal antibody against M982 TbpB. In addition, M982TbpB N-lobe retains binding activity to human transferrin conjugated to horseradish peroxidase as shown in figure 8. The results show that a fusion comprising of an oleosin and M982 TbpB N-lobe can be expressed and targeted onto the surfaces of oil bodies of oil seed plants and that M982 TbpB N-lobe retains binding activity.

In addition, the present invention also used transgenic oil bodies expressing *β*-glucuronidase (GUS) as an oleosin fusion in *Brassica napus.* In this experiment, a fusion protein comprising the GUS (beta-glucuronidase) enzyme and a oleosin gene was used for immunizations. The recombinant gene was inserted into plant cells, and transgenic plants obtained (Kuhnel, B., L. A. Holbrook, M. M. Moloney, and G. J. H. van Rooijen. 1996. Oil bodies of transgenic Brassica napus as a source of immobilized beta-glucuronidase. JAOCS 73:1533-1538). The resultant transgenic *Brassica napus* produces oil-bodies with the GUS enzyme on the surface of the oil body. Transgenic oil bodies expressing GUS were isolated as described in example 1 and used to immunized animals.

### Example 6. Immunization of animals using oi1-body-antigen complexes.

In this example, groups of female Balb/C mice (3 to 6 weeks of age) were immunized with oil-body-antigen complexes. The mice received two intraperitoneal injections of the antigen preparations two weeks apart and serum samples were obtained weekly by tail bleeds. Dilutions of the sera were analyzed for anti-TbpB antibodies by ELISA (enzyme linked immunosorbent assay) using immobilized recombinant TbpB. Bound antibodies were detected with an anti-murine IgG. (gamma-specific) conjugate and appropriate substrate. The curves were compared to that obtained with a murine IgG standard of known concentration and anti-TbpB mouse sera from the VSA3-immunized mice (pooled) in order to determine the concentration of specific antibody in the sera. Mice (n=3/group) were injected with one of the following preparations:
(i) 10 µg of recombinant TbpB,
(ii) 10 µg of recombinant TbpB in 1:4 VSA3 adjuvant/saline (VSA3 is an optimal adjuvant used for experimental veterinary vaccination experiments *e.g*., Harland, R. J., et al. 1992. The effect of subunit or modified live bovine herpesvirus-1 vaccines on the efficacy of a recombinant Pasteurella haemolytica vaccine for the prevention of respiratory disease in feedlot calves. Can. Vet. J. 33:734-741),
(iii) 10 µg of recombinant TbpB protein coupled to a biotinylated oil-body preparation containing 200 µg of oleosin (1.25:100 molar ratio),
(iv) 10 µg of recombinant TbpB coupled to a biotinylated oil-body preparation containing 20 µg of oleosin (12.5:100 molar ratio),
(v) 10 µg of recombinant TbpB in an uncoupled oil-body preparation containing 200 µg of oleosin, and
(vi) 10 µg of recombinant TbpB in an uncoupled oil-body preparation containing 20 µg of oleosin.

The immune response of the animals, as measured by specific antibody levels to the TbpB are shown.

**Table I: Antibody levels following immunization with TbpB antigen and oil-bodies**

| Immunogen | Anti-TbpB (µg/ml) | | | |
|---|---|---|---|---|
| | Week 1 | Week 2 | Week 3 | Week 4 |
| (i) TbpB alone | 21 | 39 | 750 | 1262 |
| (ii) TbpB plus VSA3 | 50 | 124 | 4471 | 2480 |
| (iii) TbpB coupled to oil bodies | 154 | 79 | 2685 | 2834 |
| (iv) TbpB coupled to oilbodies² | 142 | 71 | 3056 | 2290 |
| (v) TbpB - mix¹ | 149 | 78 | 926 | 990³ |
| (vi) TbpB - mix^{1,2} | nd | 34 | nd | 554³ |

| | | | | |
|---|---|---|---|---|
| ¹ Oil-body preparation using biotin to prevent coupling of antigen/SA to biotinylated oil bodies. ² With a molar ratio of TbpB to oleosin of approximately 1/100 instead of 1/10. ³ Responses significantly lower than model oil-body preparation by Student's T test. nd - not determined. | | | | |

The results demonstrate that the oil-body-streptavidin-TbpB complex provide a substantial increase in antibody response to TbpB compared to using the TbpB alone. Comparing the results to those obtained with 1:4 VSA3 suspension as adjuvant indicate that the oil-body-TbpB-TbpB complex provides a similar response at four weeks. The results also demonstrate that the results with a lower ratio of biotinylated antigen to biotinylated oil-body do not reduce the immune response against antigen and may provide a greater adjuvant effect.

### Example 7. Safety of oil-bodies in systemic immunization

In order for oil-bodies to be useful as a vaccine delivery system, administration of oil-bodies should not cause any undesired side effects such as acute toxicity or, an adverse immune response. The parenteral (systemic) route of administration was chosen as the most likely to cause acute toxicity. The oil-bodies were prepared under sterile conditions essentially as described above. The final fat pad was re-suspended in sterile saline to a final protein concentration of 20 mg/ml. An aliquot of the suspension was subjected to SDS-PAGE analysis to confirm the purity of the oil body preparation.

Rabbits were selected as the appropriate model as rabbits have been used for vaccine toxicity studies previously. The anticipated dosage required vaccine applications was based on the expectation that the dose of antigen would be between 2 and 50µg per injection for rabbits and that between 1 and 5% of the oleosin would be a fusion protein a transgenic seed. For a 50 kDa antigen, an anticipated vaccine dose (2 - 50 µg), would correspond to 0.4 -10 µg of oleosin in the fusion protein Thus an effective immunization dose would be 40µg -1 mg total protein (oleosin) at 1% expression and 8µg to 200 µg at 5% expression. Thus, 20 mg dose of oleosin per injection would represent a 20 -100 fold higher dose than immunization. Eight healthy, adult female New Zealand white rabbits (approximately 2.5 - 3 kg) were injected with oil-bodies intramuscularly in the thigh (1 ml containing 20 mgs of oil-body protein) and subcutaneously in the dorsal neck area (1 ml containing 20 mgs of oil-body protein) on days 0, 14 and 28. Three control rabbits were injected with 1 ml normal saline in the same regions using the same schedule. The rabbits were monitored for body temperature and general state of health daily for the duration of the experiment. After the third injection, the rabbits were sacrificed and tissue samples were taken for histopathological analysis.

The treated rabbits did not develop any increase in body temperature (relative to control animals) or any physical signs of distress (change in fur texture, etc.). Histopathological analysis of the liver, spleen, heart and muscle did not reveal any pathophysiological changes. There were no residual pathophysiological features (i.e. inflammatory infiltrate, scarring etc.) at the sites of injection. The results indicate that there are no acute signs of toxicity due to the systemic administration of plant-derived oil bodies at doses considerably higher than would ever be used for immunological purposes. In addition, the results demonstrate that there are no local or systemic pathophysiological changes from systemic administration of oil bodies.

To determine if there was an immune response to oleosin and see if this response interfered or reduced the response to specific antigens, sera from the mice in the Example 6 described above were tested for the antibody response to oleosin. Native oil-bodies were immobilized on hydrophobic protein-binding microtitre plates and ELISA performed as described above. Anti-oleosin antibody levels were calculated by comparison to the known murine IgG standard. Results are shown below.

**Table II. Oleosin antibody levels following immunization with oil-bodies**

| Immunogen | Anti-Oleosin (µg/ml) | |
|---|---|---|
| | Week 2 | Week 4 |
| (i) TbpB alone | 0 | 0 |
| (ii) TbpB plus VSA3 | 0 | 0 |
| (iii) TbpB coupled to oil bodies | 13 | 68 |
| (iv) TbpB coupled to oilbodies² | 25 | 84 |
| (v) TbpB - mix¹ | 6 | 67 |
| (vi) TbpB - mix^{1,2} | 12 | 73 |

| | | |
|---|---|---|
| ¹ Oil body preparation using biotin to prevent coupling of antigen/SA to biotinylated oil bodies. ² With a molar ratio of TbpB to oleosin of approximately 1/100 instead of 1/10. | | |

These results show that the anti-oleosin antibody response is low, does not vary much regardless of the 10 fold increase in the dose for some groups over others (groups iv and vi over iii and v) and that the anti-oleosin response does not adversely affect the specific response to the specific antigen (TbpB responses in Example 6). Similarly, a low but non-interfering response was seen to streptavidin in mice that received antigen-coupled oil-bodies or antigen-streptavidin (results not shown). The animals treated with oil-bodies (either as a control or coupled to antigen) showed no morbidity (including no evidence of acute or delayed allergic response) or mortality. By comparison, initial experiments where a 1:3 VSA3 suspension was used resulted in 100% mortality in the treated mice. Accordingly the VSA3 adjuvant is not suitable for widespread use.

### Example 8. Immunization of animals with more than one antigen.

In this example, multiple antigens were used in combination with coupling to oil-bodies. The C-terminal subfragment of tetanus toxoid (TTC) was used since it had been shown previously that the C-terminal subfragment was devoid of toxin activity yet retains its immunological properties and can be expressed as a recombinant protein in *E*: *coli* (Halpern, J. L., W. H. Habig, E. A. Neale, and S. Stibitz. 1990. Cloning and expression of functional fragment C of tetanus toxin. Infect Immun. 58:1004-1009). The C-terminal fragment was cloned by PCR, inserted into the pT7biohistag vector and recombinant antigen purified as described in example 2. The TbpB antigen was also used. Antigens were coupled to biotinylated oil-bodies as described in example 3. In the experiment examining multiple antigens, groups of mice were immunized intraperitoneally 2 weeks apart with one of the following preparations:
(i) 10 µg of recombinant TTC coupled to a biotinylated oil-body preparation containing 200 µg of oleosin (n=4),
(ii) 10 µg of recombinant TTC and 10 µg of recombinant TbpB coupled to a biotinylated oil-body preparation containing 200 µg of oleosin (n=4).
(iii) 10 µg of recombinant TbpB coupled to a biotinylated oil-body preparation containing 200 µg of oleosin (n=4).

Serum samples were obtained biweekly by tail bleeds. Anti-TTC or anti-TbpB antibody levels were determined by ELISA using immobilized recombinant TTC or TbpB and appropriate standards as describe above. The results from the immunization experiments evaluating immunization with multiple antigens compare model oil-bodies containing TTC or TbpB alone to oil-bodies with both antigens. The results demonstrate that model oil-body preparations containing more than one antigen do not compromise the response against the individual antigens. In fact, the immune response against the individual antigens was increased when both antigens are present. The results are as shown.

**Table III: Antibody levels following immunization with multiple antigens**

| Immunogen | Specific Ab Level (µg/ml) | | | |
|---|---|---|---|---|
| | anti-TTC | | anti-TbpB | |
| | Week 2 | Week 4 | Week 2 | Week 4 |
| TTC coupled to oil-bodies | 59 | 690 | <3 | <3 |
| TbpB coupled to oil-bodies | <3 | <3 | 48 | 404 |
| TTC/TbpB coupled to oil-bodies | 52 | 930 | 497 | 1476 |

### Example 9. Immunization with a oil-body preparation containing an oleosin recombinant fusion.

In this experiment, an oil body preparation from a transgenic plant expressing the beta-glucuronidase (GUS) enzyme fused to oleosin was used for immunizations. A recombinant gene encoding oleosin and GUS, was inserted into plant cells, and transgenic plants obtained (Kuhnel, B., L. A. Holbrook, M. M. Moloney, and G. J. H. van Rooijen. 1996. Oil bodies of transgenic Brassica napus as a source of immobilized beta-glucuronidase. JAOCS 73:1533-1538). The resultant transgenic *Brassica napus* produces oil-bodies with the GUS enzyme on the surface of the oil-body. Another source of recombinant GUS enzyme was obtained by the use of the bacterial expression vector pT7BHGus that can express GUS enzyme in bacteria. The expressed GUS enzyme also contains the biotinylation peptide sequence and the polyhistidine tag, allowing for purification of the recombinant enzyme and coupling of the enzyme to biotinylation oil-bodies. The vector map of pT7BHGus is shown in figure 7. In the experiment using GUS as a model antigen, groups of mice were immunized by intraperitoneal injection 2 weeks apart with one of the following preparations:
(i) 10 µg of recombinant GUS (n=3),
(ii) 10 µg of recombinant purified GUS and 3 mg/ml (0.6 mg/dose) of alum (aluminum phosphate) (n=2),
(iii) 10 µg of recombinant biotinylated GUS in a coupled oil-body preparation containing 200 µg of oleosin (n=4),
(iv) a transgenic oil body preparation containing 200 µg of oleosin and approximately 10 µg of GUS (each n=4).

Serum samples were obtained biweekly by tail bleeds. Anti-GUS antibody levels were determined by ELISA using immobilized recombinant GUS and appropriate standards as describe above. The results of the experiment demonstrate that the response was similar between the oil-bodies where the recombinant antigen is produced as a fusion product with oleosin and where the antigen was coupled to the oil-bodies by the use of biotinylation.

**Table IV: Antibody levels following immunization with GUS protein**

| Immunogen | Anti-GUS (µg/ml) | |
|---|---|---|
| | Week 2 | Week 4 |
| GUS | 4 | 38 |
| GUS plus alum | 8 | 97 |
| GUS coupled to oil-body | 13 | 153 |
| GUS transgenic oil-body | 17 | 159 |

Both oil-body preparations provide a substantial increase in antibody response to GUS compared to GUS alone. Comparing the results to those obtained with alum as adjuvant indicate that oil-bodies are a more effective adjuvant than alum. The results also demonstrate that the results with transgenic antigen oil-bodies are similar to those obtained with coupled antigen oil-bodies, indicating that the coupled antigen oil-bodies are functionally similar to the transgenic oil-bodies in systemic immunization experiments.

### Example 10: Efficacy of plant oil-bodies as a delivery vehicle for mucosal immunization (prime and prime/boost).

In order to evaluate the efficacy of mucosal administration of antigen, the intranasal route of immunization was used because it has been shown to be an effective site for mucosal immunization and does not face the same set of problems as oral immunizations. The oral/gastric route of administration was tested for comparison. The transferrin binding protein B (TbpB) from *Neisseria meningitidis* was used as the antigen and the cholera toxin beta subunit (CTB) was included as a potential targeting/immunomodulating protein to determine if coupling this protein to oil-bodies would enhance the immune response attained by mucosal immunization. For the mucosal preparations, all components were assembled as described in examples 2 and 3 and the coupled oil-bodies were concentrated by centrifugation to reduce the volume and increase the consistency.

For the addition of CTB, it proved difficult to obtain workable quantities of biotinylated protein using the pT7BioHis standard expression system. Thus an alternative expression system was employed. This alternative system utilized the pMalc2 vector (Riggs, P. 1994. Expression and purification of maltose binding protein fusions., p. 16-6-1-16-6-14. In: F. M. Ausubel, R. Brent, R. E. Kingston, D. D. Moore, J. G. Seidman, J. A. Smith, and K. Struhl (eds.), Current Protocols in Molecular Biology. Wiley, New York.). The pMalc2 vector allows expression in *E*. *coli,* similar to the pT7BioHis vector, but also contains a maltose binding protein sequence at the N-terminus of the recombinant protein. The pMalc2 vector was modified to contain the biotinylation and polyhistidine coding regions contained in the pT7BioHis vector essentially as described in example 2. The modified pMalc2 vector is referred to as pMalc2BioHis and provides a substitute for the pT7BioHis vector as a means to express recombinant proteins. The cholera toxin beta subunit gene was PCR amplified with *Taq* polymerase from a clinical isolate of *Vibrio cholera.* The PCR product contained *Xmn*I and *Hind*III sites that allowed easy insertion into the pMalc2BioHis vector. The entire construct thus contained a recombinant coding region comprising the cholera toxin beta subunit, a biotinylation consensus peptide region, a polyhistidine region and a maltose binding protein region. This expression system was induced and the biotinylated recombinant antigen was produced. This protein could be readily isolated with metal chelate chromatography and could be coupled to biotinylated oil bodies with streptavidin.

In the mouse experiments using TbpB as a model antigen, biotinylated oil bodies coupled with TbpB or with TbpB plus CTB were used. Groups of 2-3 mice were immunized with one of the following preparations and routes. Sera were collected biweekly and tested for anti-TbpB antibodies as described above.

**Table V: Routes Used for Mucosal Immunization**

| Primary Dose (TbpB-coupled oil-body preparation) | Route | w or w/o 100 g CTB | Secondary Dose (TbpB-coupled oil-body preparation) | Route | w or w/o 100 g CTB |
|---|---|---|---|---|---|
| 50 µg/1000 µg | in | w/o | 10 µg/200 µg | ip | w/o |
| 50 µg/1000 µg | in | w/o | 50 µg/1000 µg | in | w/o |
| 50 µg/1000 µg | in | w | 10 µg/200 µg | ip | w/o |
| 50 µg/1000 µg | in | w | 50 µg/1000 µg | in | w |
| 50 µg/1000 µg | ig | w/o | 10 µg/200 µg | ip | w/o |
| 50 µg/1000 µg | ig | w/o | 50 µg/1000 µg | ig | w/o |
| 50 µg/1000 µg 50 µg/1000 µg | ig | w | 10 µg/200 µg | ip | w/o |
| | ig | w | 50 µg/1000 µg | ig | w |

in - intranasal; ig - gastric (via intragastric tube); ip - intraperitoneal

The results from the mouse experiments demonstrated a relatively low systemic Anti-TbpB antibody response when the coupled biotinylated oil-body preparations were delivered by the intranasal (3 µg/ml at 4 weeks) or intragastric route (5 µg/ml at 4 weeks). The response was substantially enhanced when CTB was included in the oil-body preparations (64 and 7.3 µg/ml for intranasal and intragastric routes, respectively). Although mucosal administration of oil-bodies did not induce substantial levels of systemic antibody (prime and boost), it enhanced the immune response to subsequent parenteral immunization (259 and 139 µg /ml systemic IgG for in and ig, compared to 37 µg /ml 2 weeks after ip immunization). This indicates that the mucosal immunizations had effectively primed the immune system for subsequent parenteral immunization.

### Example 11: Efficacy of plant oil-bodies as a delivery vehicle for transdermal immunization (prime and prime/boost).

This example demonstrates that antigen coupled oil-bodies applied transdermally results in an enhanced immune response against the test antigen. Since transdermal administration is more likely to produce an enhanced mucosal immune response, evaluation of both systemic and mucosal antibody production was conducted. Transdermal immunization in combination with systemic administration was tested to see whether transdermal immunization could effectively prime the immune system even if its induction of systemic or mucosal antibody was limited. The production of recombinant antigen and preparation of antigen-coupled oil-bodies is essentially as described in Examples 2 & 3. To provide an oil-body preparation suitable for transdermal application, the antigen-coupled oil-bodies were suspended in a minimal volume of buffer so that the consistency of the oil-body suspension was like a cream or lotion. A higher dose of antigen (100 µg) and oil bodies (2 mg) were chosen for transdermal applications than with systemic administration. For transdermal application the antigen coupled oil-body preparations were applied to a 2 cm² shaved region on the back/neck/shoulder region of the mice. After application of the preparations, the mice were individually restrained in a custom device for 1 hour to prevent access to the back region. The mouse experiments used TbpB as a model antigen. Groups of 2-3 mice were immunized 2 weeks apart with one of the following preparations:
(i) 100 µg of recombinant TbpB (transdermal),
(ii) 100 µg of recombinant TbpB coupled to a biotinylated oil-body preparation containing 2 mg of oleosin (transdermal), or
(iii) 10 µg of recombinant TbpB (ip boost)
(iv) 10 µg of recombinant TbpB coupled to a biotinylated oil-body preparation containing 200 µg of oleosin (ip boost).
(v) Control groups included mice immunized with oil-bodies alone or PBS.

The mice were immunized by the transdermal or intraperitoneal route at week 0 and week 2 (as indicated below) and serum antibodies assessed at weeks 2, 4 and 8.

**Table VI: Antibody levels following transdermal immunization with TbpB**

| Immunogen | Route of Immunization | Anti-TbpB (µg/ml) | | |
|---|---|---|---|---|
| | | Week 2 | Week 4 | Week 8 |
| TbpB | td/td | <3 | 5 | 3 |
| | td/ip | 3 | 241 | 58 |
| | ip/ip | 19 | 928 | 345 |
| TbpB-coupled oil-bodies | td/td | 4 | 11 | 69 |
| | td/ip | <3 | 445 | 131 |
| | ip/ip | 37 | 1096 | 1241 |

The results from these mouse experiments demonstrate that the model oil-body preparations delivered by the transdermal route provide a low but detectable systemic (serum IgG) antibody response in contrast to administration of TbpB alone. It was particularly interesting to note that the serum antibody titer continued to rise up to 6 weeks after the last application (week 8 sample), suggesting that a more prolonged or sustained stimulation of the immune system was occurring. It clearly indicates that this route of administration shows considerable promise and that varying the timing and number of applications may provide an opportunity to further optimize the immune response.

In addition, transdermal administration of model oil bodies appeared to prime the immune system for subsequent parenteral immunization. Thus the response at Week 4 for the td/ip group (445 µg /ml) was substantially greater than that for the ip/ip group at Week 2 (37 µg /ml), both of which correspond to 2 weeks after the first parental immunization.

### Example 12: Efficacy of plant oil-bodies as a delivery vehicle for mucosal and transdermal booster immunization.

From the previous example, it appears that it may be valuable to examine combinations of routes of administration that might have useful application. For example, transdermal immunization may prove to be an effective means of boosting an initial parenteral immunization, which may have considerable appeal for human vaccine applications. To address this approach, groups of Balb/C mice (n=5) were given a primary subcutaneous (sc) injections of recombinant GUS protein in alum (10 µg GUS). Boosts followed at 4, 6, and 8 weeks using GUS-coupled oil-bodies (100µg GUS, 2 mg oleosin) by either the intranasal or transdermal routes as described above. Sera were collected at weeks 4, 6, 8, and 11 following the sc primary immunization and tested for systemic anti-GUS antibodies by ELISA as described above.

**Table VII: Antibody levels following intranasal/transdermal boosts with GUS protein**

| | | Anti-GUS in µg/ml | | | |
|---|---|---|---|---|---|
| Immunogen | Route | Week 4 | Week 6 | Week 8 | Week 11 |
| GUS-coupled oil bodies | sc/td | 75 | 75 | 98 | 195¹ |
| GUS-coupled oil bodies | sc/in | 75 | 36 | 63 | 121¹ |
| GUS | sc/td | 75 | 2 | 6 | 6² |
| GUS | sc/ in | 75 | 2 | 17 | 2² |
| GUS | sc/- | 75 | 9 | 17 | 24* |

| | | | | | |
|---|---|---|---|---|---|
| SC on week 0 followed by td or in boosts on weeks 4, 6, and 8. 1 Significantly increased over *GUS sc/-. 2 Not significantly lower than *GUS sc/-. | | | | | |

These data suggest antigen-coupled oil-bodies may be particularly useful for booster immunizations following a systemic primary immunization by the presently approved adjuvant alum. The usefulness of antigen-coupled oil-bodies for use as a human booster vaccine strategy has several potential advantages. Transdermal or intranasal routes are non-invasive compared to systemic administration, thus patient compliance can be achieved more readily, particularly with parental concerns about the more invasive booster injections. In addition, the easy of application, particularly of the transdermal formulation, may lead to self-administration of boosters via a take-home lotion or moist patch for application at set dates. This would greatly reduce costs to the health care system overall by negating several visits to physician offices for boosters. Finally, such a vaccine formulation may be given repeatedly over a longer period, which may lead to higher antibody levels because of the sustained exposure to antigen.

## Claims

1. A method for the production of an oil-body antigen complex capable of eliciting an immune response in an animal comprising:
i) isolating and purifying of native oil bodies from oilseed plants;
ii) modifying the oil bodies to provide a means to attach the antigen;
iii) preparing an antigen with a linker to provide a means to attach to the oil body;
iv) modifying the linker to permit attachment to the oil body; and
v) coupling of antigen and oil bodies,
wherein modification of the oil body is biotinylation of a protein expressed on the oil body and wherein said antigen is also biotinylated and said biotinylated oil body is bound to said biotinylated antigen by streptavidin or avidin.

2. A method according to claim 1, wherein said oilseed plant is selected from a group consisting of plants from the genus *Brassica,* plants from the genus *Linum,* plants from the genus *Glycine,* plants from the genus *Carthamus,* and plants from the genus *Arabidopsis.*

3. A vaccine comprising the oil-body antigen complex prepared in accordance with the method of claim 1 or claim 2.

4. An oil-body antigen complex prepared in accordance with the method of claim 1 or claim 2 for use as a vaccine.

## Patentansprüche

1. Verfahren zur Herstellung eines Ölkörper-Antigen-Komplexes, welcher in der Lage ist, eine Immunantwort in einem Tier auszulösen, wobei das Verfahren folgendes umfaßt:
i) Isolieren und Reinigen von nativen Ölkörpern aus Ölsamenpflanzen,
ii) Modifizieren der Ölkörper, um ein Mittel zum Anbinden des Antigens bereitzustellen,
iii) Herstellen eines Antigens mit einem Linker, um ein Mittel zum Anbinden des Ölkörpers bereitzustellen,
iv) Modifizieren des Linkers, um eine Anbindung an den Ölkörper zu gestatten, und
v) Verbinden von Antigen und Ölkörpern,
wobei die Modifikation des Ölkörpers eine Biotinylierung eines auf dem Ölkörper exprimierten Proteins ist und wobei das Antigen ebenfalls biotinyliert wird und der biotinylierte Ölkörper mittels Streptavidin oder Avidin an das biotinylierte Antigen gebunden wird.

2. Verfahren nach Anspruch 1, wobei die Ölsamenpflanze aus einer Gruppe ausgewählt ist, bestehend aus Pflanzen der Gattung *Brassica,* Pflanzen der Gattung *Linum,* Pflanzen der Gattung *Glycine,* Pflanzen der Gattung *Carthamus* und Pflanzen der Gattung *Arabidopsis.*

3. Impfstoff, welcher den Ölkörper-Antigen-Komplex, hergestellt gemäß dem Verfahren nach Anspruch 1 oder Anspruch 2, umfaßt.

4. Ölkörper-Antigen-Komplex, hergestellt gemäß dem Verfahren nach Anspruch 1 oder Anspruch 2, zur Verwendung als ein Impfstoff.

## Revendications

1. Procédé pour la production d'un complexe de corps huileux et d'antigène capable d'engendrer une réponse immunitaire chez un animal, comprenant :
i) l'isolement et la purification de corps huileux naturels à partir de plantes oléagineuses ;
ii) la modification des corps huileux pour fournir un moyen de fixation de l'antigène ;
iii) la préparation d'un antigène avec un agent de liaison pour fournir un moyen de fixation au corps huileux ;
iv) la modification de l'agent de liaison pour permettre la fixation au corps huileux ; et
v) le couplage de l'antigène et des corps huileux
dans lequel la modification du corps huileux est la biotinylation d'une protéine exprimée sur le corps huileux et dans lequel ledit antigène est également biotinylé et ledit corps huileux biotinylé est lié audit antigène biotinylé par la streptavidine ou l'avidine.

2. Procédé suivant la revendication 1, dans lequel ladite plante oléagineuse est choisie dans un groupe consistant en plantes du genre *Brassica,* plantes du genre *Linum,* plantes du genre *Glycine,* plantes du genre *Carthamus* et plantes du genre *Arabidopsis.*

3. Vaccin comprenant le complexe de corps huileux et d'antigène préparé suivant le procédé de la revendication 1 ou de la revendication 2.

4. Complexe de corps huileux et d'antigène préparé suivant le procédé de la revendication 1 ou de la revendication 2, destiné à être utilisé comme vaccin.


### SEQUENCE LISTING

<110> SEMBIOSYS GENETICS INC
<120> The Use of Plant Oil-Bodies in Vaccine Delivery Systems
<130> P015935EP
<160> 2
<170> PatentIn version 3.0
<210> 1
   <211> 89
   <212> DNA
   <213> Artificial
<220>
   <223> pT7BioHis
<400> 1
   atgctgaacg acatcttcga agctcagaaa atcgaatggc atgcccatca ccatcaccat 60
   cacgcgcatg cagctgccat ggaaagctt
   89
<210> 2
   <211> 29
   <212> PRT
   <213> Artificial
<220>
   <223> pT7BioHis
<400> 2
   Met Leu Asn Asp Ile Phe Glu Ala Gln Lys Ile Glu Trp His Ala His
   1 5 10 15
   His His His His His Ala His Ala Ala Ala Met Glu Ser
   20 25